# EUROPÄISCHE PATENTSCHRIFT

(11) **EP 3 865 176 B1**
(45) Veröffentlichungstag und Bekanntmachung des Hinweises auf die Patenterteilung: **27.09.2023**
(21) Anmeldenummer: 20157325.0
(22) Anmeldetag: 14.02.2020
(51) Int. Cl.: A61N 5/10, A61B 90/98

(54) **IDENTIFIKATION VON APPLIKATOREN FÜR EINE STRAHLENTHERAPIE**
IDENTIFICATION OF APPLICATORS FOR RADIATION THERAPY
IDENTIFICATION DES APPLICATEURS POUR UNE RADIOTHÉRAPIE

(43) Veröffentlichungstag der Anmeldung: 18.08.2021
(73) Patentinhaber: Siemens Healthcare GmbH, 91052 Erlangen (DE)
(72) Erfinder: Nioutsikou, Elena, 91056 Erlangen (DE); Requardt, Martin, 90425 Nürnberg (DE); Schneider, Manuel, 91054 Erlangen (DE)

(56) Entgegenhaltungen:
- EP-A1- 3 031 494
- DE-A1-102013 109 853
- DE-A1-102014 204 381
- DE-A1-102016 006 203
- US-A1- 2015 327 949
- US-A1- 2016 030 768
- US-A1- 2016 073 925

## Beschreibung

Die Erfindung betrifft einen Applikator zur Positionierung einer Strahlenquelle in einem Patienten, wobei der Applikator eine Kodierung aufweist, welche mittels eines medizinischen Bildgebungsverfahrens erfassbar ist. Die Erfindung betrifft ferner ein Verfahren zur Identifikation eines Applikators in einer Zielregion in einem Körper des Patienten unter Verwendung einer Magnetresonanzvorrichtung.

Bei einer Brachytherapie werden Strahlenquellen, welche radioaktive Substanzen umfassen, in einen Körper eines Patienten eingebracht, um lokal im Körper des Patienten Zielgewebe, beispielsweise Tumoren, durch Strahlung zu schädigen oder zu zerstören. Dabei kann die Strahlenbelastung für gesundes Gewebe des Körpers minimiert werden, weil die Strahlenquellen radioaktive Substanzen mit einer kurzen Strahlungsreichweite, beispielsweise Betastrahler oder Gammastrahler geringer Energie, enthalten können. Weiterhin erfolgt die Bestrahlung lokal von innerhalb des Körpers und muss nicht, wie beispielsweise bei einer externen Strahlentherapie mittels eines Linearbeschleunigers, erst von außerhalb des Körpers durch gesundes Gewebe zum Zielgewebe durchdringen.

Üblicherweise wird eine Strahlenquelle zumindest mittels eines Applikators, welcher z. B. als eine Hohlnadel und/oder ein Katheter ausgebildet sein kann, in den Körper eingeführt und in der Nähe des Behandlungsgebietes platziert. Für die Brachytherapie können beispielsweise permanent in den Körper implantierte Strahlenquellen verwendet werden. Diese permanent implantierten Strahlenquellen - auch als Kapseln oder Seeds bezeichnet - werden typischerweise mittels des Applikators in der Nähe des Zielgewebes implantiert. Ein Verfahren mit einer permanent implantierten Strahlenquellen weist üblicherweise eine niedrige Dosisleistung unter 2 Gray pro Stunde auf und wird als Low-Dose-Rate-Brachytherapie (LDR-Brachytherapie) bezeichnet. Alternativ können die Strahlenquellen mittels eines Applikators auch temporär in den Körper des Patienten eingeführt und anschließend entfernt werden. Bei dieser Art der Brachytherapie werden vorzugsweise hohe Dosisleistung, typischerweise größer als 12 Gray pro Stunde, verwendet, um ein entartetes Gewebe lokal zu bestrahlen. Dieses Verfahren wird auch High-Dose-Rate-Brachytherapie (HDR-Brachytherapie) genannt.

Zur Unterstützung der Positionierung des Applikators werden vorzugsweise bildgebende Verfahren, wie z. B. Computertomographie, Ultraschall oder Magnetresonanztomographie, eingesetzt. Während die Magnetresonanztomographie einen exzellenten Weichgewebekontrast aufweist und damit eine hohe Auflösung des Gewebes im Behandlungsgebiet erlaubt, lässt sich die Spitze des Applikators üblicherweise besser mit Hilfe der Computertomographie darstellen. Häufig werden daher mehrere bildgebende Verfahren eingesetzt, um die Positionierung eines Applikators im Körper des Patienten zu überwachen. Dies verursacht einen hohen organisatorischen und operativen Aufwand und kann aufgrund der notwendigen Kombination von Bilddaten mehrerer Bildgebungsverfahren fehleranfällig sein.

Abhängig von der Position und der Größe von Metastasen kann zudem eine hohe Anzahl von Applikatoren - z. B. bis zu 28 Applikatoren bei einer Prostata-Behandlung - erforderlich sein. In solchen Fällen kann eine Differenzierung der einzelnen Applikatoren in den Bilddaten der bildgebenden Verfahren schwierig sein, sodass die Gefahr besteht, dass Strahlenquellen an einen falschen Applikator angeschlossen werden. Dadurch können Schäden in gutartigem Gewebe entstehen und der Behandlungserfolg des Verfahrens reduziert werden.

Für eine Brachytherapie Behandlung wird, insbesondere wenn der zumindest ein Applikator in den Körper implantiert ist, typischerweise ein Bestrahlungsplan erstellt. Dieser Bestrahlungsplan sieht zum Beispiel vor, wie lange und/oder wie oft bei einer HDR-Brachytherapie die Strahlenquellen in der Nähe des Zielgewebes verweilen sollen. Alternativ oder zusätzlich kann der Bestrahlungsplan auch eine Empfehlung für eine Implantation weiterer Applikatoren, insbesondere bei einer LDR-Brachytherapie, vorsehen. Selbstverständlich kann der Bestrahlungsplan auch weitere, dem Fachmann als sinnvoll erscheinende, Maßnahmen vorsehen.

Die Druckschrift US 2016/0030768 offenbart einen Katheter, welcher bei einer Magnetresonanzbildgebung einen Kontrast bereitstellt. Der Katheter umfasst eine Lösung aus Salz und ein Kontrastmittel, wobei die Lösung in einem Abschnitt eines Katheterlumens eingeschlossen ist.

Die Druckschrift EP 3 031 494 A1 offenbart ein Verifikationssystem mit einem Kontrollelement und einem Verifikationselement zur Bestätigung einer Position einer Leitung in einem Patienten, wobei das Verifikationselement dazu ausgelegt ist, einen Referenzmarker zu detektieren und ein Signal auszugeben, welches auf eine Relativposition des Referenzmarkers und des Verifikationselements hinweist.

Bei der Druckschrift US 2016/0073925 A1 wird ein Apparat mit einer Magnetresonanztomographievorrichtung zur Aufnahme eines Bilds eines medizinischen Instruments bereitgestellt, wobei das medizinische Instrument ein Markierungsmaterial aufweist, welches eine Kernspinresonanz außerhalb einer Protonenresonanz besitzt, und wobei der Apparat dazu ausgelegt ist, eine Kernspintomographie bei der Kernspinresonanz des Markierungsmaterials auszuführen.

Die Druckschrift DE 10 2014 204 381 A1 offenbart ein Verfahren zur Planung einer Brachytherapie, wobei eine Position eines Applikators anhand von ersten Magnetresonanz-Bilddaten extrahiert wird und ein Bestrahlungsplan unter Verwendung der extrahierten Position des Applikators erfolgt.

Der Erfindung liegt daher die Aufgabe zugrunde, einen Applikator bereitzustellen, welcher sich aus einer Vielzahl von Applikatoren unterscheiden lässt und dessen Position in einfacher Weise mittels üblicher Bildgebungsverfahren überwacht werden kann.

Die Aufgabe wird durch einen erfindungsgemäßen Applikator nach Anspruch 1 und ein Verfahren nach Anspruch 8 gelöst.

Der erfindungsgemäße Applikator zur Positionierung einer Strahlenquelle in einem Patienten weist einen Tubus auf. In einer medizinischen Anwendung kann der Applikator z. B. in einem Patienten implantiert sein, um eine Bestrahlung eines malignen oder entarteten Gewebes mittels einer Strahlenquelle vorzunehmen. Ein Tubus kann z. B. als dünnwandiger Zylinder oder Röhre ausgeführt sein. Dies kann bedeuten, dass der Tubus eine Wandstärke von weniger als einem Millimeter, weniger als einem halben Millimeter und insbesondere weniger als einem Viertel Millimeter aufweist. Der Tubus kann weiterhin einen geringen Durchmesser und ein gewebeverträgliches Material aufweisen. Der Durchmesser des Tubus ist dabei insbesondere auf einen Durchmesser der Strahlenquelle abgestimmt, welche üblicherweise als zylinderförmige Kapsel ausgeführt ist. Ein Durchmesser des Tubus ist beispielsweise kleiner als acht Millimeter, kleiner als vier Millimeter oder kleiner als 2 Millimeter. Der Tubus besitzt vorzugsweise eine Längserstreckungsrichtung, welche entlang der Längsachse des Tubus orientiert ist. Der Tubus erstreckt sich dabei im Wesentlichen geradlinig entlang dieser Längserstreckungsrichtung. Als geradlinig können dabei Strukturen angesehen werden, deren Erstreckung maßgeblich in eine bestimmte Richtung weisen. Ein Tubus kann dabei ebenso Bögen oder Krümmungen entlang der Längserstreckungsrichtung aufweisen.

Der erfindungsgemäße Applikator wird vorzugsweise bei einer HDR-Brachytherapie eingesetzt, bei welcher die Strahlenquelle nicht im Körper des Patienten abgelegt wird, sondern entlang des Tubus bewegt wird, um ein umliegendes Gewebe zu bestrahlen. Eine Spitze des Tubus kann in einer Zielregion in einem Körper des Patienten positioniert sein. Unter dem Begriff Spitze wird in diesem Zusammenhang ein in Richtung des Patienten weisendes Ende des Tubus mit beliebiger Gestalt verstanden. Die Spitze ist bei einer HDR-Brachytherapie vorzugsweise geschlossen, um eine unbeabsichtigte Ablage der hochenergetischen Strahlenquelle im Körper des Patienten zu vermeiden. Es ist jedoch ebenso vorstellbar, dass der Tubus eine offene Spitze aufweist, um Strahlenquellen mit niedriger Dosisrate im Rahmen einer LDR-Brachytherapie in einer Zielregion des Patienten abzulegen. Der Tubus des erfindungsgemäßen Applikators weist vorzugsweise ein Material auf, welches sich mittels eines medizinischen Bildgebungsverfahrens von dem umliegenden Gewebe unterscheiden lässt. Ein medizinisches Bildgebungsverfahren kann z. B. eine Magnetresonanztomographie, eine Computertomographie ein Ultraschallverfahren oder dergleichen sein.

Der Tubus des Applikators ist dazu ausgebildet, die Strahlenquelle in eine Zielregion in dem Körper des Patienten zu leiten. Der Tubus weist hierfür vorzugsweise an einem außerhalb des Patienten liegenden Endes eine Öffnung auf, sodass eine Strahlenquelle in den Tubus eingeführt werden kann. Häufig verwendete Strahlenquellen sind Radionuklide wie z. B. ¹³⁷Cs, ⁶⁰Co, ¹⁹²Ir, ¹²⁵I, ¹⁰³Pd oder ¹⁰⁶Ru.

Der Applikator weist weiterhin ein Indikatorelement auf, welches mittels eines medizinischen Bildgebungsverfahrens erfassbar ist, wobei das Indikatorelement mit einem Führungselement verbunden ist und mittels des Führungselements entlang einer Längserstreckung des Tubus in einem Inneren des Tubus verschiebbar ist, wobei das Indikatorelement aus Silikon gefertigt ist und wobei das Führungselement eine Kodierung aufweist, welche mittels eines medizinischen Bildgebungsverfahrens erfassbar ist und einen ersten Applikator von einem zweiten Applikator unterscheidet.

Bei einer Brachytherapie wird häufig eine Vielzahl von Applikatoren eingesetzt, um mehrere Tumore und/oder Metastasen in einer Behandlungssitzung zu bestrahlen. Die Zielregionen können dabei verschiedene Volumina oder Abmessungen aufweisen und erfordern eine individuell angepasste Bestrahlung. Eine Anpassung der Bestrahlung kann z. B. eine Dosisleistung der eingesetzten Strahlenquelle und/oder eine Verweildauer der eingesetzten Strahlenquelle in einem Abschnitt des Tubus umfassen. Mittels der Kodierung lässt sich ein erster Applikator von einem zweiten Applikator oder auch einer Vielzahl von weiteren Applikatoren unterscheiden. Dadurch lässt sich die Strahlenquelle einem Applikator in der Zielregion des Patienten zweifelsfrei zuordnen.

Die Kodierung weist vorzugsweise ein Material auf, welches mittels eines bildgebenden Verfahrens abgebildet werden kann. Es ist auch vorstellbar, dass ein Material der Kodierung für das Bildgebungsverfahren unsichtbar ist, aber eine Information über die Position in Abhängigkeit einer Wechselwirkung mit dem umliegenden Gewebe und/oder einer veränderten Sichtbarkeit des umliegenden Gewebes erhalten werden kann. Die Kodierung kann ebenso als Gravur oder Aussparung in einem Material des Applikators ausgeführt sein, welche sich mittels eines medizinischen Bildgebungsverfahrens abbilden lässt.

Der erfindungsgemäße Applikator weist ein Indikatorelement auf, welches mittels eines medizinischen Bildgebungsverfahrens erfassbar ist. Anhand des Indikatorelements lässt sich eine Position des Applikators in der Zielregion des Patienten darstellen und überwachen. Das Indikatorelement kann einen Körper mit beliebiger Geometrie, wie z. B. ein Zylinder, ein Würfel, eine Kugel, eine Pyramide oder beliebige homöomorphe Ausprägungen dieser Köper darstellen. Das Indikatorelement kann z. B. an der Spitze des Tubus positioniert sein, um einen Ort der Ablage und/oder eine Bewegungstrajektorie der Strahlenquelle entlang des Tubus zu bestimmen. Die Bewegungstrajektorie kann z. B. anhand einer Ausrichtung des Indikatorelements und/oder einer relativen Position des Indikatorelements zum Tubus und/oder einer relativen Position des Indikatorelements zu der Kodierung bestimmt werden. Es ist ebenso vorstellbar, dass die Position des Indikatorelements an mehreren Stellen entlang der Längserstreckung des Tubus mit einem bildgebenden Verfahren erfasst wird. Dadurch lässt sich eine Position des Applikators in Relation zum umliegenden Gewebe und/oder eine Bewegungstrajektorie der Strahlenquelle zu bestimmen. Das Indikatorelement weist vorzugsweise ein Material auf, welches mittels eines bildgebenden Verfahrens erfasst werden kann. Es ist auch vorstellbar, dass ein Material des Indikatorelements für das Bildgebungsverfahren unsichtbar ist, aber eine Information über die Position in Abhängigkeit einer Wechselwirkung mit dem umliegenden Gewebe und/oder einer veränderten Sichtbarkeit des umliegenden Gewebes erhalten werden kann. Das Material des Indikatorelements unterscheidet sich vorzugsweise von dem Material der Kodierung, um eine Differenzierung der beiden Komponenten des Applikators in den Bilddaten zu ermöglichen. Mögliche Materialien des Indikatorelements stellen z. B. Metalle, Metalloxide oder Kunststoffe dar. Vorzugsweise ist das Indikatorelement aus einem Kunststoff, wie z. B. Silikon oder Nylon, gefertigt. Erfindungsgemäß ist das Indikatorelement aus Silikon gefertigt.

Das Indikatorelement ist mit einem Führungselement verbunden und mittels des Führungselements entlang einer Längserstreckung des Tubus in einem Inneren des Tubus verschiebbar. Ein Führungselement kann z. B. als eine Faser, eine Schnur, ein Faden, ein Strang oder dergleichen ausgeführt sein, welcher mit dem Indikatorelement verbunden ist. Das Führungselement kann z. B. mittels einer Schweißverbindung, einer Klebeverbindung, einer Schraube, einer Klemme oder einer sonstigen form- oder kraftschlüssigen Verbindung an dem Indikatorelement befestigt sein. Es ist weiterhin vorstellbar, dass das Indikatorelement einstückig mit dem Führungselement verbunden ist. Das Führungselement ist insbesondere dazu ausgelegt, das Indikatorelement zur Überwachung einer Position des Applikators bis in die Spitze des Tubus zu verschieben und anschließend wieder zurückzuführen. Das Führungselement kann z. B. einen ovalen oder vieleckigen Querschnitt aufweisen. Daneben kann das Führungselement auch mehrere Fäden oder Fasern aufweisen, geflochten sein oder ein Gewinde aufweisen. Der Querschnitt des Führungselements ist vorzugsweise kleiner als der Querschnitt des Tubus, um Reibungseffekte bei einer Verschiebung des Indikatorelements entlang der Längsachse des Tubus zu reduzieren. Eine mögliche Größenordnung beträgt weniger als 90 % des Querschnitts des Tubus. Vorzugsweise beträgt der Querschnitt des Führungselements weniger als 60 % des Querschnitts des Tubus. Besonders wünschenswert ist ein Querschnitt von maximal 30 % des Querschnitts des Tubus. Das Führungselement ist weiterhin dazu ausgebildet, das Indikatorelement bis in die Spitze des Tubus zu führen. Die Länge des Führungselements ist dabei vorzugsweise größer als die Länge des Tubus, sodass ein Teil des Führungselements aus dem Tubus herausragt, wenn das Indikatorelement an der Spitze des Tubus positioniert ist.

Durch das Indikatorelement kann die Position des Tubus in Relation zu einem Gewebe der Zielregion auf vorteilhafte Weise mittels eines medizinischen Bildgebungsverfahrens bestimmt und überwacht werden. Die Bilddaten des Bildgebungsverfahrens lassen sich anschließend an eine Planungseinheit für die Brachytherapie übertragen. Dadurch können die Planung und die Qualität der Strahlenbehandlung verbessert werden. Das Indikatorelement lässt sich mittels des Führungselements auf vorteilhafte Weise innerhalb des Tubus bewegen. Dadurch lassen sich beliebige Stellen entlang des Tubus mittels eines Bildgebungsverfahrens erfassen und eine Bewegungstrajektorie der Strahlenquelle bestimmen.

Durch die Kodierung lässt sich ein erster Applikator auf vorteilhafte Weise von einem zweiten Applikator unterscheiden. Dadurch kann eine Verwechslung von Applikatoren und somit eine fehlerhafte Dosierung der Strahlung für ein Gewebe vermieden werden.

Weitere vorteilhafte Ausführungsformen sind in den abhängigen Ansprüchen angegeben.

In einer Ausführungsform des erfindungsgemäßen Applikators weisen die Kodierung und/oder das Indikatorelement und/oder das Führungselement eine Wechselwirkung mit magnetischen Feldern auf und sind mittels einer Magnetresonanzvorrichtung erfassbar. Die Kodierung, das Indikatorelement werden im Folgenden auch als Komponenten des Applikators bezeichnet. Der Begriff Komponenten ist dabei nicht als abschließend zu interpretieren und kann beispielsweise weitere Bauteile oder Bestandteile des Applikators umfassen. Eine Wechselwirkung mit magnetischen Feldern kann bedeuten, dass eine Komponente des Applikators in eine Raumrichtung magnetisierbar ist, ein Magnetfeld aufweist und/oder ein elektromagnetisches Wechselfeld absorbiert und/oder emittiert. Eine Magnetresonanzvorrichtung ist üblicherweise dazu ausgebildet, ein im Wesentlichen transversal zu einem magnetischen Hauptfeld B0 ausgerichtetes, magnetisches Wechselfeld B1 zu empfangen, um eine Information über die Zusammensetzung eines Untersuchungsobjektes zu erhalten. Das magnetische Wechselfeld B1 wird dabei z. B. durch präzedierende Wasserstoffkerne (Protonen) generiert, welche durch Radiofrequenzpulse aus der Richtung des Hauptmagnetfelds B0 in Richtung des transversalen Magnetfelds B1 ausgelenkt werden. Es ist vorstellbar, dass eine Komponente des Applikators ein Material aufweist, welches Protonen in gebundener oder elementarer Form enthält und mittels eines solchen Radiofrequenzpulses angeregt werden kann. Eine Magnetresonanz-Eigenschaft des verwendeten Materials unterscheidet sich dabei vorzugsweise von einer Magnetresonanz-Eigenschaft des Gewebes der Zielregion, sodass eine Komponente des Applikators mittels der Magnetresonanztomographie von dem umliegenden Gewebe unterschieden werden können. Vorstellbare Materialien sind z. B. Kunststoffe auf Basis von Kautschuk, Silikon, Nylon oder dergleichen. Es ist ebenso vorstellbar, dass die Kodierung und/oder das Indikatorelement ein Material aufweisen, welches ein Magnetresonanzsignal lokal auslöscht, sodass es durch eine Signalauslöschung von einem umliegenden Gewebe unterscheidbar ist. Solche Materialien können z. B. magnetische, insbesondere ferro- oder ferrimagnetische Eigenschaften aufweisen. Daneben kommen auch Kontrastmittel, wie z. B. Gadolinium-Verbindungen sowie Maghemit- oder Magnetit-Nanopartikel, in Frage, welche ein Magnetresonanzsignal des umliegenden Gewebes in positiver oder negativer Weise beeinflussen können.

Durch die Verwendung magnetresonanzaktiver Materialien lässt sich der Applikator auf vorteilhafte Weise mittels einer Magnetresonanztomographie erfassen. Dadurch können der Einsatz weiterer Bildgebungsverfahren vermieden und der organisatorische und operative Aufwand einer Brachytherapie reduziert werden.

In einer weiteren Ausführungsform des erfindungsgemäßen Applikators weist das Führungselement eine Markierung auf. Eine Markierung kann z. B. eine farbliche Kennzeichnung, ein aufgefädelter oder aufgeschraubter Körper mit beliebiger Geometrie, sowie ein Knoten, ein Klebepunkt oder dergleichen darstellen, welcher auf dem Führungselement positioniert ist. Es ist auch vorstellbar, dass das Führungselement an der Position der Markierung eine Querschnittserweiterung oder ein Anschlagselement aufweist, um eine Verschiebung des Indikatorelements entlang der Längsachse des Tubus zu begrenzen. Die Markierung kann dabei auch ein Material aufweisen, welches mittels eines medizinischen Bildgebungsverfahrens erfassbar ist. Mögliche Bildgebungsverfahren stellen dabei insbesondere Magnetresonanztomographie, Computertomographie, Röntgenverfahren, Ultraschallverfahren und dergleichen dar. Anhand der Markierung ist eine Position des Indikatorelements entlang der Längserstreckung des Tubus erkennbar. Hierfür wird eine Relativposition zwischen der Markierung und einem distalen Ende des Tubus bestimmt, welche eine Information über die Position des Indikatorelements innerhalb des Tubus liefert. Unter einem distalen Ende wird in diesem Zusammenhang ein Abschluss oder eine Mündung des Tubus verstanden, welcher in entgegengesetzter Richtung zur Spitze orientiert ist. In einem einfachen Beispiel lässt sich anhand der Relativposition zwischen der Markierung und dem Ende des Tubus überprüfen, ob das Indikatorelement an der Spitze des Tubus positioniert ist. Es ist aber ebenso vorstellbar, dass das Führungselement mehrere Markierungen aufweist, anhand derer verschiedenen Positionen des Indikatorelements innerhalb des Tubus eingestellt und/oder überprüft werden können.

Durch den Einsatz einer Markierung lässt sich auf vorteilhafte Weise eine ordnungsgemäße Position des Indikatorelements innerhalb des Tubus überprüfen. Der Einsatz einer Querschnittserweiterung oder eines Anschlagselements kann die Einstellung der Position des Indikatorelements im Tubus erleichtern und die Handhabung des Applikators vereinfachen.

Gemäß einer weiteren Ausführungsform des erfindungsgemäßen Applikators weisen der Tubus und/oder das Indikatorelement und/oder das Führungselement eine Kodierung aus einem vorbestimmten Material auf. Die Kodierung ist dabei vorzugsweise mit dem Tubus und/oder dem Indikatorelement und/oder dem Führungselement verbunden. Die Verbindung ist vorzugsweise so ausgeführt, dass eine Ablösung des Materials der Kodierung bei einer anwendungsgemäßen Verwendung des Applikators vermieden wird. Die Verbindung kann beispielsweise durch Kleben, Schweißen, Pressen oder dergleichen erfolgen. Es ist auch vorstellbar, dass das Material der Kodierung in die Oberfläche des Tubus und/oder des Indikatorelements und/oder des Führungselements eingebettet ist. Eine solche Einbettung kann z. B. durch Imprägnieren der Oberfläche mit dem Material der Kodierung erfolgen. Es ist aber auch ein Auffüllen einer geprägten oder geätzten Struktur auf der Oberfläche vorstellbar. Die Kodierung kann weiterhin auch als "Perlenschnur" ausgeführt sein, wobei eine vorbestimmte Abfolge von Knoten und/oder Querschnittserweiterungen in dem Führungselement eine Kodierung darstellt. Hierbei können z. B. ein Abstand und/oder eine Abmessung der Knoten oder Querschnittserweiterungen zur Kodierung einer Information herangezogen werden. Eine Information stellt insbesondere einen Identifikator oder eine Kennung dar, welche einen ersten Applikator von einem zweiten Applikator unterscheidbar macht. Die Kodierung ist vorzugsweise mittels eines medizinischen Bildgebungsverfahrens, wie z. B. eines Magnetresonanztomographen, eines Computertomographen oder eines Ultraschallgeräts erfassbar.

In einer möglichen Verkörperung ist die Kodierung in der Oberfläche des Tubus des Applikators eingebettet. Auf diese Weise lässt sich der Tubus nach einer Implantation in der Zielregion des Patienten jederzeit mittels eines medizinischen Bildgebungsverfahrens identifizieren und von anderen Applikatoren unterscheiden. In einem anderen Beispiel weist das Indikatorelement eine Kodierung auf. Auf diese Weise lässt sich der Applikator bei der Positionierung des Indikatorelements in dem Tubus identifizieren und in den Bilddaten registrieren. Das Indikatorelement kann anschließend aus dem Tubus entfernt werden, um eine Strahlenquelle aufzunehmen.

Durch die Kodierung einer Komponente des Applikators lässt sich ein Applikator in einer Zielregion mit einer Mehrzahl von Applikatoren im Körper des Patienten auf vorteilhafte Weise unterscheiden. Dadurch können Fehler bei der Bestückung einzelner Applikatoren mit Strahlenquellen vermieden werden.

In einer weiteren Ausführungsform ist das Material der Kodierung eines Applikators als ein individuelles Muster ausgebildet, welches über ein medizinisches Bildgebungsverfahren erfassbar ist, wobei das Muster einteilig oder aus disjunkten Abschnitten zusammengesetzt ist. Die Kodierung kann, wie oben beschrieben, mit dem Tubus und/oder dem Indikatorelement und/oder dem Führungselement des Applikators verbunden und/oder in diese eingebettet sein. Das Material der Kodierung ist dabei so auf einer oder mehrerer der genannten Komponenten des Applikators angeordnet, dass ein individuelles Muster entsteht. Ein individuelles Muster kann z. B. vorbestimmte Zeichen und/oder Symbole sowie Folgen von Zeichen und/oder Symbolen umfassen, welche eine Information über den Applikator kodieren. Mögliche Beispiele für ein solches Muster sind beliebige Arten von Strichcodes, Punktcodes oder Matrixcodes, welche Informationen anhand eines Abstands, einer Abfolge, einer Anordnung und/oder einer Abmessung entsprechender Zeichen kodieren. Ein individuelles Muster kann auf einem begrenzten Abschnitt des Applikators, z. B. der Spitze, dem Ende oder der Mitte des Tubus positioniert oder über die gesamte Längserstreckung des Applikators verteilt sein. Vorzugsweise ist die Kodierung auf einem proximalen Abschnitt des Applikators positioniert, welcher sich in der Zielregion des Patienten befindet und somit in einem Bildgebungsbereich des Bildgebungsverfahrens liegt.

Es ist ebenso vorstellbar, dass die Kodierung aus einzelnen oder mehreren Abstrakten Symbolen besteht, welche durch eine spezifische Form, eine spezifische Materialstärke und/oder ein spezifisches Material differenzierbar sind. Bei einem einteiligen Muster kann das Muster eine einzelne Figur aufweisen. Diese Figur kann eine beliebig komplexe Kontur aufweisen und sich dadurch von Figuren anderer Applikatoren unterscheiden. Es ist ebenso vorstellbar, dass sich die Figur aus mehreren disjunkten Abschnitten zusammensetzt, welche sich gegeneinander nicht überlagern.

In einem Beispiel ist das medizinische Bildgebungsverfahren ausgebildet, ein Muster eines Applikators mittels eines Bildverarbeitungsalgorithmus automatisch zu erkennen und das Muster zu dekodieren. Eine Information über den Applikator kann anschließend in den Bilddaten gespeichert und auf einer Anzeigeeinheit einem Nutzer zur Verfügung gestellt werden.

Durch die Verwendung eines grafischen Musters zur Kodierung von Informationen lässt sich eine Vielzahl von Informationen über den Applikator kodieren. Diese Informationen können auf vorteilhafte Weise auf einer Komponente des Applikators im Bildgebungsbereich der Bildgebungsvorrichtung positioniert sein. Die Verwendung grafischer Muster ermöglicht zudem eine automatische Erkennung und Dekodierung mittels eines medizinischen Bildgebungsverfahrens und erleichtert somit den Umgang mit einer Vielzahl von Applikatoren.

Das erfindungsgemäße System umfasst einen ersten Applikator mit einer ersten Kodierung und einen zweiten Applikator mit einer zweiten Kodierung, wobei der erste Applikator und der zweite Applikator anhand der ersten Kodierung und der zweiten Kodierung mittels eines medizinischen Bildgebungsverfahrens unterscheidbar sind. Die erste Kodierung und die zweite Kodierung können nach einem der oben genannten Beispiele ausgeführt sein. Die erste Kodierung kann sich beispielweise bezüglich eines Musters, einer Zeichenkette oder eines Abstands zwischen einzelnen Zeichen oder Mustern von der zweiten Kodierung unterscheiden. Dieser Unterschied lässt sich mittels eines der oben genannten medizinischen Bildgebungsverfahren abbilden und z. B. mittels eines Bildverarbeitungsalgorithmus automatisch erkennen. Auf diese Weise können der erste Applikator und der zweite Applikator eindeutig in den Bilddaten des medizinischen Bildgebungsverfahrens zugeordnet werden. Es ist vorstellbar, dass jeder Applikator bei der Fertigung mit einer individuellen Kodierung versehen wird, sodass der erste Applikator eine von dem zweiten Applikator und jedem weiteren Applikator verschiedene Kodierung aufweist. Es ist ebenso vorstellbar, dass ein System eine maximale Anzahl von beispielsweise 30, 40 oder 50 Applikatoren aufweist, welche sich bezüglich der Kodierung voneinander unterscheiden und mit der medizinischen Bildgebungsvorrichtung registriert werden können. Die Anzahl an unterschiedlichen Kodierungen auf den Applikatoren entspricht in diesem Fall vorzugsweise der maximalen Anzahl von Applikatoren, welche sich mit der medizinischen Bildgebungsvorrichtung registrieren lassen. Bei dieser Ausführung können einzelne Applikatoren bei einem Defekt gegen Applikatoren mit identischer Kodierung ausgetauscht werden, ohne dass eine erneute Registrierung mit der medizinischen Bildgebungsvorrichtung notwendig ist.

Durch den Einsatz eines Systems mit einer vorbestimmten Anzahl von Applikatoren mit unterschiedlicher Kodierung lassen sich der Aufwand der Herstellung und der Registrierung der Applikatoren mit einem medizinischen Bildgebungsverfahren auf vorteilhafte Weise reduzieren.

Gemäß dem erfindungsgemäßen Verfahren zur Identifikation eines Applikators zur Positionierung einer Strahlenquelle in einer Zielregion in einem Körper eines Patienten unter Verwendung einer Magnetresonanzvorrichtung weist der Applikator einen Tubus auf und ist ausgebildet, eine Strahlenquelle entlang des Tubus in die Zielregion zu leiten. Eine Zielregion kann, wie oben beschrieben, z. B. ein Tumor, eine Metastase oder eine beliebige Form eines entarteten Gewebes darstellen, welche mittels einer Strahlenquelle bestrahlt werden soll. Es ist vorstellbar, dass der Körper des Patienten mehrere solcher Zielregionen aufweist, welche im Rahmen einer Brachytherapie mit einem oder mehreren Applikatoren versehen sind. Die Zielregionen können dabei so nahe aneinander positioniert sein, dass mehrere Applikatoren oder Teile von Applikatoren in einen Bildgebungsbereich einer Zielregion hineinragen und sich teilweise oder vollständig überlagern. Die Applikatoren weisen individuelle Kodierungen auf, welche mit der Magnetresonanzvorrichtung erfassbar sind und die Applikatoren untereinander differenzieren. Die Kodierung kann hierfür ein Material umfassen, welches eine Wechselwirkung mit einem magnetischen Feld aufweist und/oder eine Magnetresonanz-Eigenschaft eines umliegenden Gewebes in einem Magnetresonanz-Bilddatensatz verändert. Es ist vorstellbar, dass die Magnetresonanzvorrichtung eine Bildverarbeitungseinheit aufweist, welche eine Kodierung in einem Magnetresonanz-Bilddatensatz automatisch erkennt und eine Information zur Unterscheidung der Applikatoren in den Magnetresonanz-Bilddaten einfügt, bevor diese auf einer Anzeigeeinheit der Magnetresonanzvorrichtung dargestellt werden.

In einem Schritt des Verfahrens wird mittels einer ersten Bildgebungssequenz ein erster Magnetresonanz-Bilddatensatz erfasst, wobei erste Bildgebungsparameter der ersten Bildgebungssequenz auf eine hohe Auflösung eines Gewebes in der Zielregion des Patienten gerichtet sind, wobei sich der Applikator in der Zielregion befindet. Eine erste Bildgebungssequenz kann dabei erste Bildgebungsparameter umfassen, welche u. A. eine Auflösung, einen Kontrast, ein Signal-RauschVerhältnis, einen Bildgebungsbereich sowie eine Dauer eines Hochfrequenzpulses und/oder einen zeitlichen Abstand zwischen verschiedenen Hochfrequenzpulsen bestimmen. Hierbei können beliebige Magnetresonanz-Sequenzen eingesetzt werden, welche einen ausreichenden Kontrast zur Unterscheidung von physiologischem und pathologischem Gewebe bereitstellen. Beispiele für solche Sequenzen sind Spin-Echo-Sequenzen, Multi-Echo-Sequenzen, Gradienten-Echo-Sequenzen sowie diverse Inversionstechniken, mit denen ein ausreichender Kontrast diagnostisch relevanter Strukturen möglich ist. Ein Magnetresonanz-Bilddatensatz mit einer hohen Auflösung des Zielgewebes kann dabei insbesondere eine guten Gewebekontrast, ein hohes Signal-Rausch-Verhältnis sowie einen auf die Zielregion angepassten Bildgebungsbereich aufweisen. Je nach Behandlungsstrategie und Verteilung des entarteten Gewebes in dem Körper des Patienten können Teile von einem oder mehreren Applikatoren in einen Bildgebungsbereich des ersten Magnetresonanz-Bilddatensatzes hineinragen. Die Unterscheidung der verschiedenen Applikatoren erfolgt anhand der individuellen Kodierung jedes Applikators in einem dedizierten Verfahrensschritt. Der erste Magnetresonanz-Bilddatensatz kann ferner eine Durchführung multipler Bildgebungssequenzen mit gleichen oder unterschiedlichen Bildgebungsparametern umfassen, welche auf eine hohe Auflösung des Gewebes in der Zielregion des Patienten ausgerichtet sind.

In einem weiteren Verfahrensschritt wird eine Position und eine Kennung eines Applikators in der Zielregion anhand des ersten Magnetresonanz-Bilddatensatzes bestimmt. Eine Kennung kann dabei z. B. eine Kette von Zeichen und/oder Ziffern darstellen, anhand derer sich ein individueller Applikator bestimmen lässt. Vorzugsweise weist jeder Applikator eine einzigartige Zeichenkette auf, anhand derer sich der Applikator zweifelsfrei aus einer Mehrzahl von Applikatoren bestimmen lässt. Die Bestimmung der Position und der Kennung eines Applikators in der Zielregion kann beispielweise in Abhängigkeit von Absolutwerten und/oder Relativwerten der Kontraste, der Graustufen, der Signalamplituden oder dergleichen erfolgen. Diese werden aus dem ersten Magnetresonanz-Bilddatensatz extrahiert, welcher zweidimensionale und dreidimensionale Bilddaten, aber auch Rohdaten, wie z. B. k-Raum Daten, enthalten kann. Es ist ebenso vorstellbar, dass die Kennung des Applikators kodiert ist. Durch eine Kodierung lässt sich eine Vielzahl an Informationen über den Applikator auf einem geringen Raum bereitstellen. Es ist z. B. vorstellbar, dass die Kennung des Applikators, wie oben beschrieben, als grafisches Muster kodiert ist. Die Kodierung kann aber auch mittels eines dynamischen Vorgangs, wie z. B. einer Fortbewegungsweise des Indikatorelements in dem Tubus des Applikators, implementiert sein.

In einem weiteren Schritt des erfindungsgemäßen Verfahrens erfolgt die Ausgabe der Position und der Kennung des Applikators. Dies kann bedeuten, dass eine Information über die Position und die Kennung eines Applikators zusammen mit dem ersten Magnetresonanz-Bilddatensatz abgespeichert werden. Vorzugsweise werden die Informationen jedoch zumindest an eine Anzeigeeinheit der Magnetresonanzvorrichtung übertragen, welche die Informationen über einen Applikator im Bildgebungsbereich zusammen mit dem ersten Magnetresonanz-Bilddatensatz des Gewebes der Zielregion für einen behandelnden Mediziner visualisiert. Es ist aber ebenso vorstellbar, dass die Kennung und die Position eines Applikators in einer Zielregion zusammen mit dem ersten Magnetresonanz-Bilddatensatz an eine Planungseinheit übertragen werden. Eine Planungseinheit kann z. B. dazu ausgebildet sein, die lokale Verweildauer der Strahlenquelle in dem Tubus in Abhängigkeit einer Anzahl und/oder einer Lage von Applikatoren sowie einer Größe und/oder einer Anzahl von Tumoren zu bestimmen.

Durch das erfindungsgemäße Verfahren lässt sich der herausragende Weichgewebekontrast einer Magnetresonanz-Bildgebung auf vorteilhafte Weise zur Unterstützung der Planung einer Brachytherapie einsetzen. Dadurch lässt sich der Einsatz weiterer Bildgebungsverfahren vermeiden, welche einen zusätzlichen organisatorischen und operativen Aufwand bedeuten.

In einer weiteren Ausführungsform des erfindungsgemäßen Verfahrens weist der Applikator eine Kodierung auf, welche mittels der Magnetresonanzvorrichtung erfasst wird und zur Bestimmung der Kennung und/oder der Position des Applikators in der Zielregion des Patienten herangezogen wird. Die Kodierung weist dabei vorzugsweise ein Material auf, dessen Magnetresonanz-Eigenschaften sich, wie oben beschrieben, von einem Material des Applikators sowie dem umliegenden Gewebe unterscheiden. Dadurch lässt sich die Kodierung eines Applikators von anderen Objekten im Bildgebungsbereich der Magnetresonanzvorrichtung unterscheiden. Besonders geeignete Materialien für die Kodierung stellen z. B. Lanthanoide wie Lanthan, Neodym und Gadolinium dar, welche aufgrund von paramagnetischen Eigenschaften ihrer Ionen zu einer schnelleren Relaxation von Protonen in einem umliegenden Gewebe führen und somit den Kontrast der nahegelegenen Gebiete verändern. Daneben können auch andere Materialien, wie z. B. paramagnetische oder super-paramagnetische Maghemit-Nanopartikel eingesetzt werden, welche ebenfalls eine schnellere Relaxation von Wasserstoffprotonen in der unmittelbaren Umgebung verursachen. Materialen mit solchen Eigenschaften werden häufig als positive Kontrastmittel bezeichnet, da das betreffende Gewebe in T1-gewichteten Magnetresonanz-Bilddaten heller dargestellt wird. Es ist aber ebenso ein Einsatz von ferri- oder ferromagnetischen Materialien, wie z. B. Magnetit- oder Eisenpartikeln, vorstellbar, welche ein Magnetresonanzsignal lokal abschwächen, ablenken oder auslöschen und daher in Magnetresonanz-Bilddaten dunkler erscheinen können. Durch den Einsatz positiver und/oder negativer Kontrastmittel als Material für die Kodierung lässt sich bereits aus dem ersten Magnetresonanz-Bilddatensatz, welcher auf eine hohe Auflösung des Gewebes der Zielregion ausgerichtet ist, eine Information über die Position und die Kennung des Applikators bestimmen.

Es ist vorstellbar, dass die Kodierung an einer Spitze des Tubus des Applikators positioniert ist. Die Kodierung kann den Tubus dabei auf vorbestimmten Abschnitten in einer Längserstreckung außenumfänglich umschließen. In diesem Fall lässt sich aus der Kodierung nicht nur eine Kennung des Applikators ableiten, sondern auch die Position der Spitze sowie eine Orientierung des Applikators in dem Gewebe der Zielregion.

Durch die Kodierung des Applikators mit einem magnetresonanzaktiven Material lassen sich die Position und die Kennung des Applikators auf vorteilhafte Weise bereits in Abhängigkeit des ersten Magnetresonanz-Bilddatensatzes bestimmen. Dadurch kann die zur Durchführung des Verfahrens benötigte Zeit reduziert werden.

In einer weiteren Ausführungsform des erfindungsgemäßen Verfahrens wird in einem Schritt ein Indikatorelement mittels eines Führungselements innerhalb eines Tubus des Applikators positioniert. Ein Indikatorelement kann, wie oben beschrieben, ein Körper mit einer beliebigen Geometrie sein, welcher an einem Führungselement befestigt ist. Das Indikatorelement kann aber auch einstückig mit dem Führungselement verbunden sein. Vorzugsweise weist das Indikatorelement ein Material auf, welches mit magnetischen Feldern wechselwirkt und zumindest eine Magnetresonanz-Eigenschaft aufweist, welche sich von einer Magnetresonanz-Eigenschaft des umliegenden Gewebes unterscheidet. Das Indikatorelement wird vorzugsweise vor dem Erfassen des ersten Magnetresonanz-Bilddatensatzes in dem Tubus positioniert, sodass der erste Magnetresonanz-Bilddatensatz Bildinformationen über das Indikatorelement enthält. Es ist z. B. vorstellbar, dass das Indikatorelement ein Material mit einer paramagnetischen Eigenschaft aufweist, welches eine Veränderung des Kontrasts des umliegenden Gewebes in dem ersten Magnetresonanz-Bilddatensatz verursacht. Es ist ebenso vorstellbar, dass das paramagnetische Material des Indikatorelements gleichzeitig eine Kennung des Applikators kodiert.

Das Indikatorelement kann aber auch einen der oben genannten, magnetresonanzaktiven Kunststoffe aufweisen. Bei einer solchen Ausführung kann die Positionierung des Indikatorelements auch nach dem Erfassen des ersten Magnetresonanz-Bilddatensatzes erfolgen. Vorzugsweise wird in diesem Fall eine zweite Bildgebungssequenz zur Erfassung eines zweiten Magnetresonanz-Bilddatensatzes durchgeführt, welche auf eine hohe Auflösung des Indikatorelements gerichtet ist. Es ist weiterhin vorstellbar, dass sich eine Bewegung des Indikatorelements und eine Bildgebungssequenz der Magnetresonanzvorrichtung zeitlich überlagern. Dies kann z. B. bedeuten, dass die Position des Indikatorelements während der Bewegung mehrfach mittels des Bildgebungsverfahrens erfasst wird oder die Bewegung des Indikatorelements einen Kontrastunterschied in einem Magnetresonanz-Bilddatensatz verursacht.

Durch die Positionierung des Indikatorelements in dem Tubus des Applikators lässt sich das Volumen des magnetresonanzaktiven Materials auf vorteilhafte Weise gegenüber dem dünnwandigen, Tubus des Applikators erhöhen. Dadurch können ein höheres Magnetresonanzsignal und ein besserer Kontrast des Applikators in dem ersten Magnetresonanz-Bilddatensatz erreicht werden.

In einer weiteren Ausführungsform des erfindungsgemäßen Verfahrens wird in einem Schritt ein zweiter Magnetresonanz-Bilddatensatz erfasst, wobei zweite Bildgebungsparameter einer zweiten Bildgebungssequenz auf eine hohe Auflösung des Indikatorelements gerichtet sind und das Bestimmen einer Position des Applikators in der Zielregion anhand des ersten Magnetresonanz-Bilddatensatzes und des zweiten Magnetresonanz-Bilddatensatzes erfolgt. Die Erfassung des zweiten Magnetresonanz-Bilddatensatzes erfolgt vorzugsweise nach der Positionierung des Indikatorelements in dem Tubus des Applikators. Das Indikatorelement weist bei dieser Ausführungsform vorzugsweise einen magnetresonanzaktiven Kunststoff auf, wie z. B. Kautschuk, Nylon, Silikon oder dergleichen, dessen Magnetresonanz-Eigenschaften sich von einem umliegenden Gewebe unterscheiden. Es ist jedoch vorstellbar, dass sich der Kontrast des Kunststoffs nicht ausreichend von dem umliegenden Gewebe unterscheidet. In einem solchen Fall kann die zweite Bildgebungssequenz mit zweiten Bildgebungsparametern durchgeführt werden, welche auf einen hohen Kontrast des Indikatorelements ausgerichtet ist. Eine zweite Bildgebungssequenz kann z. B. eine Silikon-gewichtete Sequenz darstellen. Bei einer Silikon-gewichteten Bildgebungssequenz wird z. B. eine Echozeit, eine Repetitionszeit, eine Anregungshäufigkeit, eine Sättigung oder dergleichen so eingestellt, dass das Silikon im Bildgebungsbereich des zweiten Magnetresonanz-Bilddatensatzes ein hohes Signal und/oder einen hohen Kontrast zum umliegenden Gewebe aufweist.

Der zweite Magnetresonanz-Bilddatensatz kann somit eine hohe Auflösung des Indikatorelements aufweisen, während der erste Magnetresonanz-Bilddatensatz auf eine hohe Auflösung des umliegenden Gewebes gerichtet ist. Durch Kombination des ersten Magnetresonanz-Bilddatensatzes und des zweiten Magnetresonanz-Bilddatensatzes, z. B. mittels Subtraktion, Überlagerung und/oder Kombination von segmentierten Bildbereichen, lassen sich das Gewebe und der Applikator in der Zielregion mit hoher Auflösung visualisieren und an eine Planungseinheit übertragen.

Durch die Erfassung eines ersten Magnetresonanz-Bilddatensatzes und eines zweiten Magnetresonanz-Bilddatensatzes lassen sich auf vorteilhafte Weise individuell angepasste Bildgebungssequenzen für das Gewebe und den Applikator durchführen, welche eine hohe Auflösung sowohl der Applikatoren als auch des Gewebes in der Zielregion des Patienten ermöglichen. Dadurch lassen sich die Planung und die Qualität der Brachytherapie verbessern. Weiterhin können Partialvolumen-Effekte in den Bilddaten durch Subtraktion des ersten Magnetresonanz-Bilddatensatzes und des zweiten Magnetresonanz-Bilddatensatzes auf vorteilhafte Weise vermieden werden.

In einer verwandten Ausführungsform des erfindungsgemäßen Verfahrens sind die zweiten Bildgebungsparameter der zweiten Bildgebungssequenz auf eine hohe Auflösung des Gewebes in der Zielregion des Patienten gerichtet. Vorzugsweise wird das Indikatorelement bei dieser Ausführungsform zwischen der Erfassung des ersten Magnetresonanz-Bilddatensatzes und des zweiten Magnetresonanz-Bilddatensatzes in dem Tubus des Applikators positioniert. Die Bilddaten des ersten Magnetresonanz-Bilddatensatzes und des zweiten Magnetresonanz-Bilddatensatzes können sich daher bezüglich eines Einflusses des Indikatorelements unterscheiden. Der Unterschied zwischen dem ersten Magnetresonanz-Bilddatensatz und dem zweiten Magnetresonanzbilddatensatz hängt dabei u. A. von einer Magnetresonanz-Eigenschaft, einer Abmessung des Indikatorelements sowie einer Wechselwirkung eines Materials des Indikatorelements mit einem umliegenden Gewebe ab. Eine Differenz zwischen dem ersten Magnetresonanz-Bilddatensatz und dem zweiten Magnetresonanz-Bilddatensatz wird zur Bestimmung einer Position des Indikatorelements herangezogen. Dies kann, wie oben beschrieben, z. B. mittels einer Subtraktion, einer Überlagerung und/oder einer Übertragung segmentierter Bildbereiche des ersten Magnetresonanz-Bilddatensatzes und/oder des zweiten Magnetresonanz-Bilddatensatzes erfolgen.

Durch die Erfassung von zwei Magnetresonanz-Bilddatensätzen, welche auf eine hohe Auflösung des Gewebes in der Zielregion des Patienten ausgerichtet sind, lassen sich auf vorteilhafte Weise zwei Bilddatensätze mit einem hohen Weichgewebekontrast aufnehmen. Dadurch können die Position eines entarteten Gewebes mit höherer Genauigkeit bestimmt und ein Signal-RauschVerhältnis der erhaltenen Bilddaten verbessert werden. Zudem kann die Dauer des Bildgebungsverfahrens reduziert werden, da die Bildgebungsparameter der Bildgebungssequenz nur einmal an die Zielregion des Patienten angepasst werden müssen.

In einer weiteren Ausführungsform des erfindungsgemäßen Verfahrens erfolgt die Bestimmung der Kennung des Applikators anhand einer Fortbewegungsgeschwindigkeit des Indikatorelements, wobei die zweite Bildgebungssequenz auf eine hohe Auflösung der Fortbewegungsgeschwindigkeit des Indikatorelements gerichtet ist. Eine Fortbewegungsgeschwindigkeit des Indikatorelements kann z. B. über das Verhältnis einer Weglänge entlang des Tubus zu einer Zeitdauer, in welcher das Indikatorelement diese Weglänge zurücklegt, charakterisiert sein. Es ist vorstellbar, dass eine Bewegung des Indikatorelements während der zweiten Bildgebungssequenz eine Phasenverschiebung der Protonen-Spins des Indikatorelements verursacht, z. B. durch Anwendung eines phasencodierenden Gradientenfelds. Um diese Phasenverschiebung der Protonen-Spins zu erfassen, kann die zweite Bildgebungssequenz als eine Phasen-Kontrast-Sequenz ausgelegt sein. Diese kann z. B. einen oder mehrere bipolare Gradienten aufweisen, welche eine Fortbewegungsgeschwindigkeit von bewegten Protonen-Spins in einer Phasenverschiebung kodieren. Die Größenordnung der Phasenverschiebung der bewegten Protonen-Spins lässt sich dabei z. B. durch eine Höhe und/oder einen Zeitpunkt eines bipolaren Gradienten beeinflussen. In einer bevorzugten Ausführungsform ist der bipolare Gradient in einer Fortbewegungsrichtung eines Indikatorelements im Tubus des Applikators ausgerichtet. Dadurch lässt sich ein empfangenes Signal der phasenverschobenen Protonen-Spins maximieren und ein Signal-RauschVerhältnis des zweiten Magnetresonanz-Bilddatensatzes verbessern.

Es ist vorstellbar, dass sich die Fortbewegungsgeschwindigkeiten mehrerer Indikatorelemente während der Aufnahme des zweiten Magnetresonanz-Bilddatensatzes unterscheiden. Die unterschiedlichen Fortbewegungsgeschwindigkeiten können zu einer unterschiedlichen Phasenverschiebung der Protonen-Spins verschiedener Indikatorelemente führen und als Kontrastunterschiede in dem zweiten Magnetresonanz-Bilddatensatz dargestellt werden. Diese Kontrastunterschiede können eine Kodierung darstellen, welche ein Indikatorelement einem Tubus zuordnen. Die Magnetresonanzvorrichtung kann beispielsweise eine Bildverarbeitungseinheit aufweisen, welche Kontrastunterschiede zwischen unterschiedlichen Indikatorelementen erfasst und den Indikatorelementen eine Kennung zuweist. Die Kennung kann z. B. in einer Datenbank vorliegen, in welcher Informationen über spezifische mit einer Fortbewegungsgeschwindigkeit assoziierte Grauwerte gespeichert sind. Es ist aber ebenso vorstellbar, dass die Fortbewegungsgeschwindigkeiten der Indikatorelemente bekannt sind, sodass ein Grauwert eines Indikatorelements in Relation zu den Grauwerten anderer Indikatorelemente direkt zur Bestimmung des korrespondierenden Applikators herangezogen werden kann.

Das Indikatorelement weist hierfür vorzugsweise ein magnetresonanzaktives Material mit molekular gebundenem Wasserstoff auf. Mögliche Materialien sind Silikon, Kautschuk, Nylon sowie beliebige Kohlenwasserstoffe.

Durch die Kodierung der Kennung eines Applikators mittels der Fortbewegungsgeschwindigkeit des Indikatorelements lässt sich ein Fertigungsaufwand der Applikatoren auf vorteilhafte Weise reduzieren. Dadurch können die Applikatoren kosteneffizienter hergestellt werden.

In einer weiteren Ausführungsform des erfindungsgemäßen Verfahrens wird in einem Schritt zumindest anhand der Position eines Indikatorelements und/oder der Kodierung des Applikators eine relative Position des Applikators zu einem Gewebe in der Zielregion bestimmt. Es ist vorstellbar, dass die Kodierung zusätzlich zur Funktion als Kennung für einen spezifischen Applikator auch eine Markierung darstellt, welche z. B. einen vorbestimmten Abstand zu einer Spitze und/oder einem Ende des Tubus kennzeichnet. Zudem kann die Kodierung, wie oben beschrieben, einen vorbestimmten Abschnitt des Tubus außenumfänglich umschließen. Dies kann eine Bestimmung der räumlichen Orientierung der Kodierung in dem ersten Magnetresonanz-Bilddatensatz erleichtern. Aus der räumlichen Orientierung der Kodierung lassen sich wiederum eine Bewegungstrajektorie, ein Winkel und/oder eine Position des Applikators in Relation zu einem Gewebe der Zielregion bestimmen. Es ist weiterhin vorstellbar, dass in Abhängigkeit des ersten Magnetresonanz-Bilddatensatzes ein dreidimensionales Bild des entarteten Gewebes in der Zielregion erstellt wird. Durch eine Berechnung der Distanz zwischen einem Referenzpunkt auf der Oberfläche des Tubus und einem Referenzpunkt auf der Oberflächenkontur des entarteten Gewebes lässt sich eine Lage des Applikators in Relation zu dem entarteten Gewebe in der Zielregion des Patienten quantifizieren und eine Strahlenwirkung der Strahlenquelle auf das umliegende Gewebe bestimmen. Der Punkt auf der Oberfläche des Tubus befindet sich vorzugsweise mittig an einer Spitze des Tubus. Es ist ebenso vorstellbar, dass mehrere Referenzpunkte in diskreten Abständen über die Oberfläche des Tubus verteilt sind. Diese Referenzpunkte können z. B. herangezogen werden, um eine mittlere Distanz zu einem Punkt auf der Oberflächenkontur des entarteten Gewebes zu bestimmen. Dabei können auch mehrere Punkte auf der Oberflächenkontur des entarteten Gewebes, sowie ein Volumenschwerpunkt, ein Massenschwerpunkt, ein Flächenschwerpunkt sowie Massenschwerpunkte mehrerer Schnitte des entarteten Gewebes als Referenzpunkte herangezogen werden. In analoger Weise lassen sich Referenzpunkte auf dem Indikatorelement heranziehen, um einen oder mehrere Abstände zu Referenzpunkten des entarteten Gewebes zu bestimmen.

Es ist ebenso vorstellbar, dass multiple erste Magnetresonanz-Bilddatensätze erfasst werden, während das Indikatorelement entlang der Längserstreckung im Inneren des Tubus bewegt wird. Dadurch lässt sich der Abstand zwischen Referenzpunkten des Indikatorelements und des entarteten Gewebes entlang der Bewegungstrajektorie des Indikatorelements in nahezu kontinuierlicher Weise bestimmen und bei der Planung der Brachytherapie berücksichtigen.

Durch die Bestimmung der Distanz zwischen Referenzpunkten entlang des Tubus und Referenzpunkten in dem entarteten Zielgewebe lässt sich die Bestrahlung des Gewebes durch die Strahlenquelle auf vorteilhafte Weise im Vorfeld quantifizieren. Dadurch kann die Strahlendosis der beweglichen Strahlenquelle für das umliegende Gewebe besser dosiert werden.

In einer weiteren Ausführungsform des erfindungsgemäßen Verfahrens wird in Abhängigkeit der Kodierung ein Modell des Applikators in dem ersten Magnetresonanz-Bilddatensatz ausgegeben. Die Kodierung des Applikators kann, wie oben beschrieben, mittels einer Bildverarbeitungseinheit automatisch erkannt und dekodiert werden. Hierfür kann die Bildverarbeitungseinheit einen Dekodierer aufweisen, welcher z. B. ein grafisches Muster der Kodierung in eine Kennung des Applikators übersetzt und mit dem ersten Magnetresonanz-Bilddatensatz verknüpft. Dies kann beispielweise bedeuten, dass ein Name, eine Nummer oder eine beliebige weitere Kennung des Applikators bei der Darstellung der Bilddaten auf einer Anzeigeeinheit der Magnetresonanzvorrichtung mit dem Applikator verknüpft ist und in einer Nähe zu dem Applikator visualisiert wird.

Der Dekodierer kann die Information über den Applikator auch an einen Prozessor der Magnetresonanzvorrichtung weiterleiten. Der Prozessor der Magnetresonanzvorrichtung kann dazu ausgelegt sein, in Abhängigkeit der Information Daten über den Applikator aus einer Datenbank zu laden. Die Daten über den Applikator können neben der Kennung z. B. auch Informationen über Abmessungen und/oder Materialien des Applikators umfassen. Insbesondere können die Daten auch eindimensionale, zweidimensionale oder vorzugsweise dreidimensionale Modelle des Applikators enthalten, welche in dem ersten Magnetresonanz-Bilddatensatz visualisiert werden können. Es ist insbesondere vorstellbar, dass eine Kodierung des Modells mit einer erfassten, räumlichen Orientierung der Kodierung in dem ersten Magnetresonanz-Bilddatensatz registriert wird. Beispielsweise wird hierbei die räumliche Lage der Kodierung eines dreidimensionalen Modells des Applikators mit der räumlichen Orientierung der erfassten Kodierung in Übereinstimmung gebracht. Das Modell des Applikators kann somit in einer korrekten Orientierung in den ersten Magnetresonanz-Bilddatensatz integriert werden. Es ist ebenso vorstellbar, dass eine Schnittdarstellung des dreidimensionalen Modells des Applikators mit den Bildgebungsparametern des ersten Magnetresonanz-Bilddatensatzes abgestimmt ist. Dadurch lassen sich beliebige Schnittebenen und Schnittwinkel des erfassten Magnetresonanzen-Bilddatensatzes mit entsprechenden Schnittebenen und Schnittwinkel des dreidimensionalen Modells des Applikators versehen und auf der Anzeigeeinheit darstellen.

Durch die Registrierung des dreidimensionalen Modells des Applikators anhand der mit dem ersten Magnetresonanz-Bilddatensatz erfassten Kodierung lässt sich die Durchführung einer zweiten Bildgebungssequenz zur Auflösung des Indikatorelements auf vorteilhafte Weise vermeiden. Dadurch kann die Dauer der bildgebenden Untersuchung reduziert werden. Zudem lässt sich die Genauigkeit der Darstellung des Applikators in dem ersten Magnetresonanz-Bilddatensatz mittels des dreidimensionalen Modells erhöhen.

Weitere Vorteile und Einzelheiten ergeben sich aus der nachfolgenden Beschreibung von Ausführungsbeispielen in Verbindung mit den Zeichnungen. Es zeigen in Prinzipdarstellung:
- Fig. 1: eine Schemadarstellung des Systems aus Magnetresonanzvorrichtung und erfindungsgemäßem Applikator,
- Fig. 2: eine mögliche Ausführungsform des erfindungsgemäßen Applikators,
- Fig. 3: eine mögliche Ausführungsform des erfindungsgemäßen Applikators,
- Fig. 4: eine mögliche Ausführungsform des erfindungsgemäßen Applikators,
- Fig. 5: ein mögliches Ablaufdiagramm des erfindungsgemäßen Verfahrens.

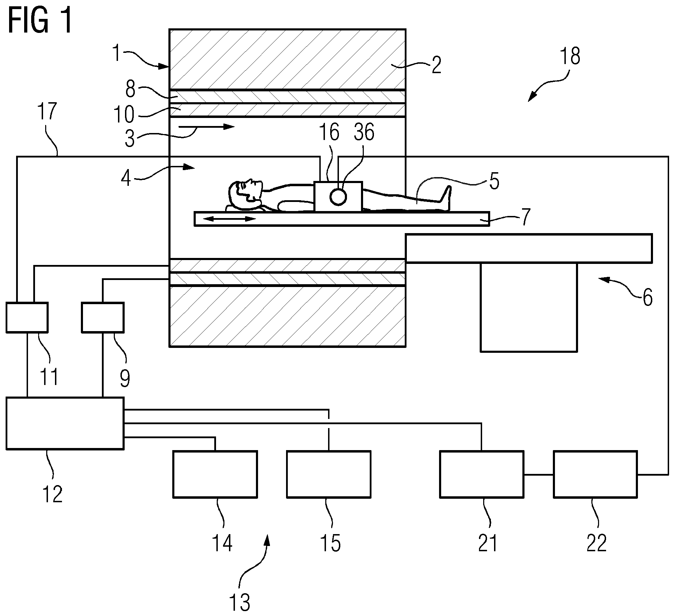

In Fig. 1 ist eine Magnetresonanzvorrichtung 18 schematisch dargestellt. Die Magnetresonanzvorrichtung 18 umfasst eine Magneteinheit 1, welche z. B. einen Permanentmagneten, einen Elektromagneten oder einen supraleitenden Hauptmagneten 2 zur Erzeugung eines starken und insbesondere homogenen Hauptmagnetfelds 3 aufweist. Zudem umfasst die Magnetresonanzvorrichtung 18 einen Patientenaufnahmebereich 4 zu einer Aufnahme eines Patienten 5. Der Patientenaufnahmebereich 4 ist im vorliegenden Ausführungsbeispiel zylinderförmig ausgebildet und in einer Umfangsrichtung von der Magneteinheit 1 umgeben. Grundsätzlich sind jedoch auch von diesem Beispiel abweichende Ausbildungen des Patientenaufnahmebereichs 4 vorstellbar. Der Patient 5 kann mittels einer Patientenlagerungsvorrichtung 6 der Magnetresonanzvorrichtung 18 in dem Patientenaufnahmebereich 4 positioniert werden. Die Patientenlagerungsvorrichtung 6 weist hierfür einen innerhalb des Patientenaufnahmebereichs 4 bewegbar ausgestalteten Patiententisch 7 auf. Die Magneteinheit 1 weist weiterhin eine Gradientenspule 8 zum Erzeugen von Magnetfeldgradienten auf, welche für eine Ortskodierung während einer Bildgebung verwendet wird. Die Gradientenspule 8 wird mittels einer Gradientensteuereinheit 9 des Magnetresonanztomographen 18 angesteuert. Die Magneteinheit 1 umfasst weiterhin eine Hochfrequenzantenne, welche im vorliegenden Ausführungsbeispiel als fest in die Magnetresonanzvorrichtung 18 integrierte Körperspule 10 ausgebildet ist. Die Körperspule 10 ist zu einer Anregung von Atomkernen ausgelegt, die sich in dem von dem Hauptmagneten 2 erzeugten Hauptmagnetfeld 3 befinden. Die Körperspule 10 wird von einer Hochfrequenzeinheit 11 des Magnetresonanztomographen 18 angesteuert und strahlt hochfrequente Signale in einen Untersuchungsraum ein, der im Wesentlichen von einem Patientenaufnahmebereich 4 der Magnetresonanzvorrichtung 18 gebildet ist. Die Körperspule 10 ist weiterhin zum Empfang von Magnetresonanzsignalen ausgebildet.

Zu einer Steuerung des Hauptmagneten 2, der Gradientensteuereinheit 9 und zur Steuerung der Hochfrequenzeinheit 11 weist die Magnetresonanzvorrichtung 18 eine Steuereinheit 12 auf. Die Steuereinheit 12 ist dazu ausgebildet, eine Durchführung einer Sequenz, wie z. B. einer bildgebenden Gradienten-Echosequenz oder einer Silikon-gewichteten Sequenz der Magnetresonanzvorrichtung 18, zu steuern. Zudem umfasst die Steuereinheit 12 eine nicht näher dargestellte Auswerteeinheit zu einer Auswertung von digitalisierten Magnetresonanzsignalen, die während der Magnetresonanzuntersuchung erfasst werden.

Des Weiteren umfasst die Magnetresonanzvorrichtung 18 eine Benutzerschnittstelle 13, welche eine Signalverbindung mit der Steuereinheit 12 aufweist. Steuerinformationen, wie beispielsweise Bildgebungsparameter und rekonstruierte Magnetresonanzbilder, können auf einer Anzeigeeinheit 14, beispielsweise auf zumindest einem Monitor der Benutzerschnittstelle 13, für einen Nutzer angezeigt werden. Weiterhin weist die Benutzerschnittstelle 13 eine Eingabeeinheit 15 auf, mittels der Informationen und/oder Parameter während eines Mess- oder Prüfvorgangs von dem Nutzer eingegeben werden können. Ferner weist die Magnetresonanzvorrichtung 18 einen Sensor, wie z. B. eine Kopfspule oder eine Lokalspule 16, auf. Eine Lokalspule 16 kann insbesondere auch eine in den Patienten eingeführte Spule sein, welche in direkter Nähe zu einem Applikator 30 in der Zielregion 36 des Patienten 5 positioniert ist und üblicherweise ein besonders hohes Signal-RauschVerhältnis aufweist. Eine mögliche Ausführungsform einer implantierten Spule ist z. B. eine endorektale Spule. Die elektrische Anschlussleitung 17 der Lokalspule 16 umfasst zumindest eine elektrisch leitfähige Leitungsstruktur. Ebenso wie die Körperspule 10 ist auch die Lokalspule 16 zu einer Anregung von Atomkernen und zum Empfang von Magnetresonanzsignalen ausgelegt und kann von der Hochfrequenzeinheit 11 angesteuert werden. Zur Signalübertragung ist die Lokalspule 16 mittels einer elektrischen Anschlussleitung 17 über die Hochfrequenzeinheit 11 mit der Steuereinheit 12 verbunden. Die Steuereinheit 12 ist zur Übertragung von Bilddaten mit der Planungseinheit 21 verbunden. Die Planungseinheit 21 ist vorzugsweise dazu ausgebildet, die Bilddaten der Magnetresonanzvorrichtung 18 zu analysieren und anhand der Bilddaten einen Bestrahlungsplan zu erstellen. Dieser Bestrahlungsplan kann z. B. eine Dosisleistung, eine Verweilzeit sowie eine Verweilposition der Strahlenquelle im Tubus 32 des Applikators 30 umfassen. Zur Durchführung der Strahlentherapie ist die Planungseinheit 21 mit einem Afterloader 22 verbunden. Der Afterloader 22 ist vorzugsweise dazu ausgebildet, eine Strahlentherapie in Abhängigkeit des Bestrahlungsplans durchzuführen. Der Afterloader 22 ist hierfür mit einem oder mehreren Applikatoren 30 verbunden und kann diese mit Strahlenquellen beladen. Der Afterloader 22 kann ebenso dazu ausgebildet sein, Indikatorelemente 34 in den Tubus 32 der Applikatoren 30 zu positionieren. Es vorstellbar, dass der Afterloader 22 hierfür unterschiedliche Magazine mit Strahlenquellen und Indikatorelementen 34 aufweist, welche sich je nach Bedarf mittels Führungselementen 31 in den Applikatoren 30 positionieren lassen. Die dargestellte Magnetresonanzvorrichtung 18 kann selbstverständlich weitere Komponenten umfassen, welche Magnetresonanzvorrichtungen üblicherweise aufweisen.

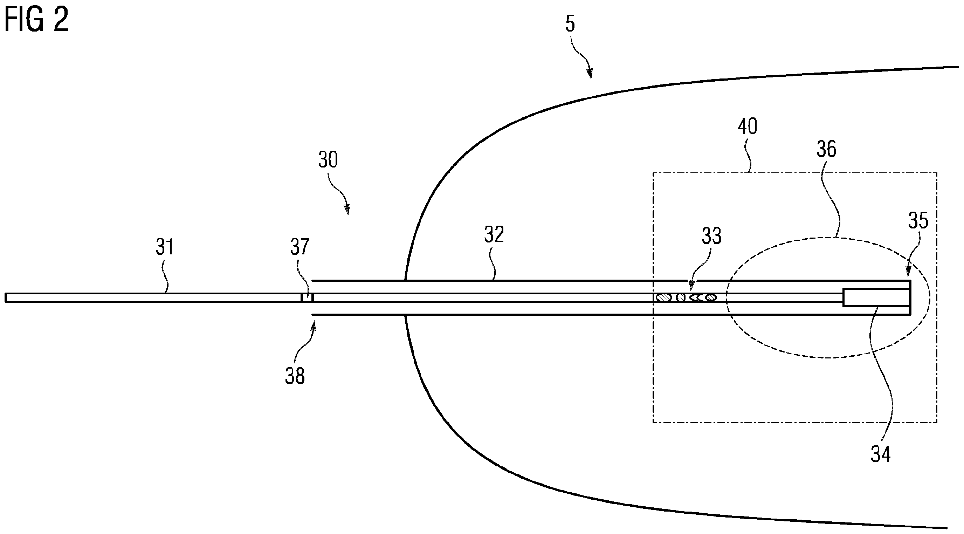

Fig. 2 zeigt eine mögliche Ausführungsform des erfindungsgemäßen Applikators 30, bei welcher der erfindungsgemäße Applikator 30 eine geschlossene Spitze 35 aufweist. Die geschlossene Spitze 35 begrenzt den Bewegungsweg einer Strahlenquelle (nicht gezeigt) sowie des Indikatorelements 34 in den Körper des Patienten 5. Das Indikatorelement 34 ist mit einem Führungselement 31 verbunden und lässt sich mit diesem innerhalb des Tubus 32 verschieben. Die Verbindung zwischen Führungselement 31 und Indikatorelement 24 kann, wie oben beschrieben, mittels Schrauben, Kleben, Schweißen oder einer beliebigen Art von form- oder kraftschlüssiger Verbindung ausgeführt sein. Das Indikatorelement 34 kann weiterhin auch einstückig mit dem Führungselement 31 verbunden sein. Der Tubus 32 ist in dem gezeigten Beispiel teilweise in dem Patienten 5 implantiert, sodass die Spitze 35 in einer Zielregion 36 positioniert ist, während das Ende 38 des Tubus 32 aus dem Patienten 5 herausragt. Eine Markierung 37 auf dem Führungselement 31 zeigt eine Relativposition zwischen dem Indikatorelement 34 und dem Tubus 32 an. Anhand der Markierung 37 lässt sich auf besonders einfache Weise beurteilen, ob das Indikatorelement 34 an der Spitze 35 des Tubus 32 positioniert ist. Sobald das Indikatorelement 34 an der Spitze 35 des Tubus 32 positioniert ist, können eine oder mehrere Bildgebungssequenzen durchgeführt werden, mit welcher sich die Position der Spitze 35 in der Zielregion 36 überprüfen lässt.

Die Kodierung 33 ist in dem dargestellten Beispiel mit dem Führungselement 31 verbunden und wird beim Zurückziehen des Indikatorelements 34 aus dem Tubus 32 aus dem Bildgebungsbereich 40 entfernt. Die Registrierung des Applikators 30 erfolgt daher vorzugsweise anhand eines geeigneten Bildgebungsverfahrens, solange sich die Kodierung 33 und das Indikatorelement 34 in dem Tubus 32 befinden. Die Kennung eines individuellen Applikators 30 kann z. B. in den Bilddaten der ersten Bildgebungssequenz registriert und abgespeichert werden, sodass der Applikator 30 in einem weiteren Verlauf der Brachytherapie identifiziert ist und die Kodierung 33 entfernt werden kann.

Da die Spitze 35 bei dieser Ausführungsform verschlossen ist, kann die Strahlenquelle nicht in der Zielregion 36 des Patienten 5 abgelegt werden. Die auf Fig. 2 dargestellte Ausführungsform wird daher vorzugsweise für eine HDR-Brachytherapie eingesetzt, bei welcher eine hochenergetische Strahlenquelle zur Bestrahlung des Gewebes in der Zielregion 36 mittels eines Führungselements 31 in dem Tubus 32 bewegt wird.

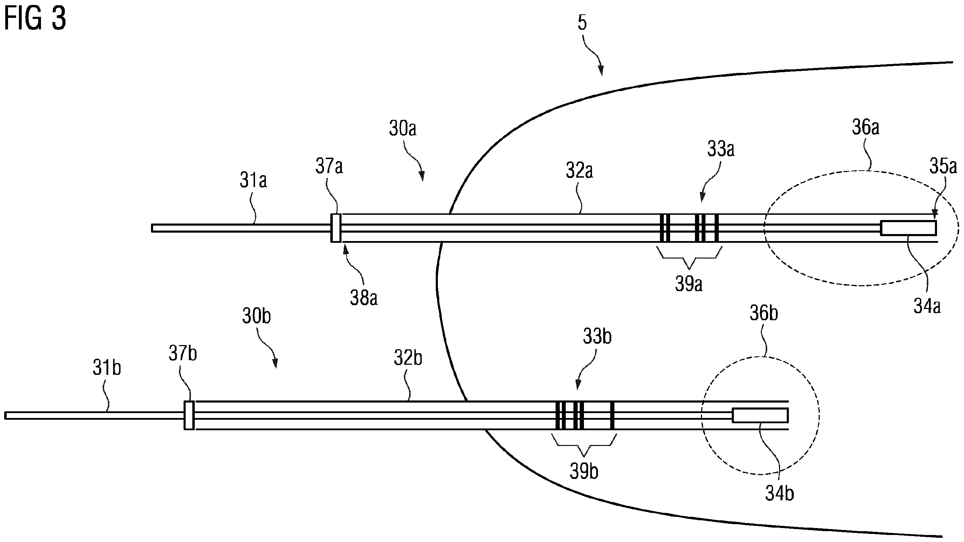

Fig. 3 zeigt eine weitere, mögliche Ausführungsform des erfindungsgemäßen Applikators 30, bei welcher ein erster Applikator 30a eine offene Spitze 35a aufweist. Diese Ausführungsform des Applikators 30a ist für eine Ablage von niedrigenergetischen Strahlungsquellen (Seeds) in der Zielregion 36a des Patienten 5 geeignet und wird vorzugsweise bei einer LDR-Brachytherapie eingesetzt. Ein weiterer Unterschied zur Ausführungsform in Fig. 2 stellt die Markierung 37a dar, welche im gezeigten Beispiel als eine Querschnittserweiterung ausgeführt ist. Die Querschnittserweiterung kann z. B. einen Knoten, einen Klebepunkt, einen eingeflochtenen Ring oder dergleichen darstellen, welche eine Bewegung des Indikatorelements 34a über die Spitze 35a des Tubus 32a hinaus verhindern. Vorzugsweise ist die Markierung 37a so auf dem Führungselement 31a positioniert, dass ein in Richtung der Spitze 35a weisendes Ende des Indikatorelements 34a mit der Spitze 35a des Tubus 32a abschließt.

Die Kodierung 33a ist in der dargestellten Ausführungsform in Form von disjunkten, konzentrischen Ringen ausgeführt, welche den Tubus 32a auf einem Abschnitt 39a außenumfänglich umschließen. Ein erster Applikator 30a und ein zweiter Applikator 30b können z. B. anhand eines Abstands und/oder einer Breite und/oder einer Anordnung der Ringe voneinander unterschieden werden. Das Material der Kodierung 33a kann dabei in die Oberfläche des Tubus 32a eingebettet sein. Besonders bevorzugte Materialien stellen z. B. Aggregate und/oder Mikrocluster von Maghemit- und/oder Magnetit-Nanopartikeln dar. Daneben sind natürlich auch weitere der oben genannten Materialien sowie Kombinationen und Legierungen daraus vorstellbar.

Der zweite Applikator 30b in Fig. 3 umfasst einen Tubus 32b, ein Führungselement 31b, ein Indikatorelement 34b, eine Kodierung 33b sowie eine Markierung 37b. Die Kodierung 33b erstreckt sich über einen Abschnitt 39b des Tubus 32b und weist eine zu der Kodierung 33a verschiedene Anordnung auf. Aufgrund der unterschiedlichen Anordnung der Kodierung 33a und der Kodierung 33b lässt sich der erste Applikator 30a mittels eines Bildgebungsverfahrens von dem zweiten Applikator 30b differenzieren. Der zweite Applikator 30b kann, wie in Fig. 3 gezeigt, in einer anderen Zielregion 36b des Patienten 5 positioniert sein. Es ist aber ebenso vorstellbar, dass der erste Applikator 30a und der zweite Applikator 30b in der gleichen Zielregion 36 des Patienten 5 positioniert sind. Neben dem ersten Applikator 30a und dem zweiten Applikator 30b können selbstverständlich noch weitere Applikatoren 30 vorliegen, welche sich anhand der Kodierung 33 unterscheiden.

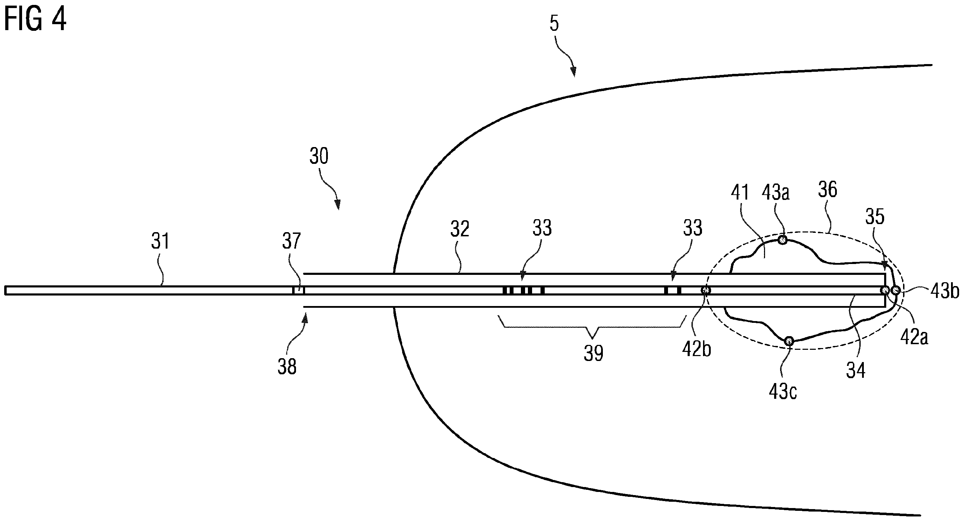

Fig. 4 zeigt eine weitere Ausführungsform des erfindungsgemä-ßen Applikators 30, bei welcher sich die Kodierung 33 über einen breiteren Bereich des Führungselements 31 erstreckt. Es ist vorstellbar, dass eine Lage und/oder Orientierung des Applikators 30 bereits anhand der Bilddaten der Kodierung 33 bestimmt werden kann, ohne dass ein Indikatorelement 34 benötigt wird. Es ist ebenso vorstellbar, dass die Kodierung 33 an dem Tubus 32 anstelle des Führungselements 31 positioniert ist. In diesem Fall kann auf einen Einsatz des Führungselements 31 zur Identifikation des Applikators 30 und einer Bestimmung seiner relativen Lage zur Zielregion 36 verzichtet werden.

Die Kodierung 33 ist im Vergleich zu Fig. 3 über einen breiteren Abschnitt des Applikators 30 verteilt. Durch die größere Längserstreckung lässt sich die Genauigkeit einer Bestimmung der Lage des Applikators 30 in Relation zur Zielregion 36 erhöhen. Es ist insbesondere vorstellbar, dass ein ein- oder mehrdimensionales Modell des Applikators 30 anhand der Kodierung 33 in den Bilddaten in Übereinstimmung gebracht und mit diesen registriert und/oder abgespeichert wird. Dadurch lässt sich ein hochauflösendes Modell des Applikators 30 in den Bilddaten visualisieren, auch wenn die restlichen Komponenten des Applikators 30 nur eingeschränkt oder gar nicht auf den Bilddaten des Bildgebungsverfahrens sichtbar sind. Dadurch lässt sich insbesondere eine weitere Aufnahme von Bilddaten vermeiden, welche lediglich auf eine Darstellung des Applikators 30 ausgerichtet sind.

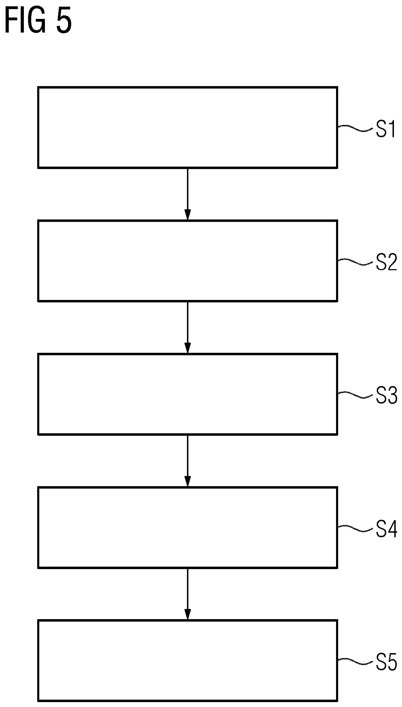

In Fig. 5 ist ein mögliches Ablaufdiagramm des erfindungsgemäßen Verfahrens dargestellt. Zu Beginn des erfindungsgemäßen Verfahrens ist der Tubus 32 vorzugsweise bereits nahe einer Zielregion 36 im Körper des Patienten 5 implantiert. Das erfindungsgemäße Verfahren wird insbesondere von einer Magnetresonanz-Bildgebung begleitet, wobei die Magnetresonanz-Bilddatensätze vorzugsweise zu einer Unterscheidung verschiedener Applikatoren 30 im Körper des Patienten 5 sowie zur Überwachung einer korrekten Position der Applikatoren 30 eingesetzt werden. Die Aufgezeichneten Magnetresonanz-Bilddatensätze können auch zur Vorbereitung einer Durchführung der Brachytherapie an eine Planungseinheit 21 und/oder eine Anzeigeeinheit weitergeleitet werden.

In einem Schritt S1 des erfindungsgemäßen Verfahrens wird das Indikatorelement 34 in das nach außen geöffnete Ende 38 des Tubus 32 eingeführt und bis an die Spitze 35 des Tubus 32 vorgeschoben. Der Transport des Führungselements 31 erfolgt dabei vorzugsweise mittels eines dafür geeigneten Geräts, wie z. B. eines Afterloaders 22. Die Bewegungsgeschwindigkeit des Indikatorelements 34 kann beliebig variieren. Sollen während der Bewegung des Indikatorelements 31 bereits erste Magnetresonanz-Bilddatensätze erzeugt werden, kann die Bewegungsgeschwindigkeit an eine Aufnahmedauer der Magnetresonanzvorrichtung 18 angepasst sein. Es ist aber auch vorstellbar, dass die erforderlichen Magnetresonanz-Bilddatensätze vor oder nach der Positionierung des Indikatorelements 34 erfasst werden. In diesem Fall kann eine möglichst kurze Dauer für die Positionierung wünschenswert sein.

In einem Schritt S2 wird ein erster Magnetresonanz-Bilddatensatz des Patienten 5 erfasst. Der erste Bildgebungsbereich 40 ist dabei so bemessen, dass eine Zielregion 36 im Körper des Patienten 5 abgebildet wird. Vorzugsweise umfasst der erste Bildgebungsbereich 40 zumindest einen wesentlichen Anteil der Zielregion 36 sowie eines Abschnitts eines oder mehrerer Applikatoren 30, die in der Zielregion 36 positioniert sind. Der erste Bildgebungsbereich 40 ist insbesondere so gewählt, dass ein Indikatorelement 34 sowie eine Kodierung 33 eines Applikators 30 in der Zielregion 36 vom ersten Bildgebungsbereich 40 umfasst sind (siehe Fig. 2). Die ersten Bildgebungsparameter der ersten Bildgebungssequenz des ersten Magnetresonanz-Bilddatensatzes sind vorzugsweise auf eine hohe Auflösung des Gewebes in der Zielregion 36 ausgerichtet. Dies kann beispielsweise bedeuten, dass ein Kontrast zwischen verschiedenen Gewebearten und einem entarteten Gewebe 41 so gewählt ist, dass eine gute Identifikation der Gewebe und ihrer Konturen durch einen behandelnden Mediziner möglich ist.

In einem Schritt S3 wird ein zweiter Magnetresonanz-Bilddatensatz des Patienten 5 erfasst. Der zweite Bildgebungsbereich entspricht dabei vorzugsweise dem ersten Bildgebungsbereich 40, sodass die Bilddaten des ersten Magnetresonanz-Bilddatensatzes und des zweiten Magnetresonanz-Bilddatensatzes einen gleichen Abschnitt des Patienten 5 abbilden. Dies bedeutet insbesondere, dass die abgebildeten Abschnitte eines oder mehrerer Applikatoren 30 sowie der Zielregion 36 des ersten Magnetresonanz-Bilddatensatzes und des zweiten Magnetresonanz-Bilddatensatzes im Wesentlichen miteinander übereinstimmen. Die zweiten Bildgebungsparameter des zweiten Magnetresonanz-Bilddatensatzes können gegenüber dem ersten Magnetresonanz-Bilddatensatz jedoch auf eine hohe Auflösung eines Applikators 30 ausgerichtet sein, welcher in der Zielregion 36 des Patienten 5 positioniert ist. Dies bedeutet insbesondere, dass eine der oben beschriebenen Bildgebungssequenzen für Kunststoff, wie z. B. einer Silikon-gewichteten Sequenz, verwendet wird. In einer Ausführungsform des erfindungsgemäßen Applikators 30 besteht auch die Kodierung 33 aus Kunststoff, welcher mittels einer entsprechenden Bildgebungssequenz für Kunststoff gut aufgelöst wird.

In einem weiteren Schritt S4 wird eine Position und eine Kennung eines Applikators 30 in der Zielregion 36 zumindest anhand des ersten Magnetresonanz-Bilddatensatzes bestimmt. Die Kennung des Applikators 30 wird dabei aus der Kodierung 33 abgeleitet, welche z. B. mittels eines Bildverarbeitungsalgorithmus aus dem ersten Magnetresonanz-Bilddatensatz extrahiert wird. Die Daten der extrahierten Kodierung 33 können anschließend an eine Dekodiereinheit weitergeleitet werden, welche die Kennung des Applikators 30 erfasst. Es ist vorstellbar, dass die Kennung anschließend mit dem entsprechenden Applikator 30 in den Bilddaten verknüpft und auf einer Anzeigeeinheit visualisiert wird. Die Kodierung 33 kann aber auch zur Anpassung der Orientierung eines dreidimensionalen Modells des Applikators 30 herangezogen werden, welches mit der Kodierung 33 in dem ersten Magnetresonanz-Bilddatensatz registriert wird.

Die Bestimmung der Lage des Applikators 30 in Relation zur Zielregion 36 des Patienten 5 kann aber auch anhand des Indikatorelements 34 erfolgen. Hierfür werden der erste Magnetresonanz-Bilddatensatz und der zweite Magnetresonanz-Bilddatensatz herangezogen, anhand derer sich eine Position des Applikators 30 bestimmen lässt. Ist der erste Magnetresonanz-Bilddatensatz auf eine hohe Auflösung des Gewebes ausgerichtet und der zweite Magnetresonanz-Bilddatensatz auf eine hohe Auflösung des Applikators, lässt sich die Position des Applikators 30 z. B. durch eine Kombination, eine Subtraktion sowie eine Segmentierung und eine Überlagerung der beiden Bilddatensätze bestimmen. Es ist aber ebenso vorstellbar, dass der zweite Magnetresonanz-Bilddatensatz, ebenso wie der erste Magnetresonanz-Bilddatensatz, auf eine hohe Auflösung des Gewebes in der Zielregion 36 des Patienten 5 ausgerichtet ist. In diesem Fall erfolgt die Positionierung des Indikatorelements 34 innerhalb des Tubus 32 vorzugsweise zwischen der Erfassung des ersten Magnetresonanz-Bilddatensatzes und des zweiten Magnetresonanz-Bilddatensatzes. Die Lage des Applikators 30 lässt sich vorzugsweise mittels eines der oben beschriebenen Verfahren aus den beiden Magnetresonanz-Bilddatensätzen bestimmen.

In einem Schritt S5 werden die Position und die Kennung eines Applikators 30 ausgegeben. Die Ausgabe der Position und der Kennung eines Applikators 30 kann z. B. über eine Visualisierung auf einer Anzeigeeinheit der Magnetresonanzvorrichtung 18 erfolgen. Hierbei wird die Kennung eines Applikators 30 z. B. als digitaler Marker oder als überlagertes Bildsegment in einem Magnetresonanz-Bilddatensatz abgespeichert und visualisiert. Die Position des Applikators 30 kann, wie im Schritt S4 beschrieben, z. B. durch eine Subtraktion des ersten Magnetresonanz-Bilddatensatzes und des zweiten Magnetresonanz-Bilddatensatzes dargestellt werden. Es ist aber ebenso vorstellbar, dass ein dreidimensionales Modell des Applikators 30 als digitales Bildelement oder durch eine Anpassung von Graustufen einzelner Pixel, welche z. B. auf dem Umriss des Applikators 30 positioniert sein können, in den Bilddaten eingebettet ist. In einer bevorzugten Ausführung wird das Modell des Applikators als separates Datenobjekt gespeichert, welches von gängigen Planungseinheiten für Brachytherapien interpretiert und zur Visualisierung auf der Anzeigeeinheit mit dem Magnetresonanz-Bilddatensatz kombiniert oder überlagert werden kann. Ein mögliches Beispiel für eine solches Datenobjekt ist eine DICOM RT Struktur.

Eine Ausgabe kann weiterhin bedeuten, dass Informationen über die Position und die Kennung eines Applikators 30 an eine Planungseinheit 21 übertragen werden, welche die Verabreichung oder den Transport von Strahlenquellen durch den Tubus 32 vorbereitet und/oder durchführt. Hierbei kann, wie in Fig. 4 gezeigt, beispielsweise eine Distanz zweier Referenzpunkte 42a und 43b herangezogen werden, um die Position des Applikators 30 in Relation zu dem entarteten Gewebe 41 zu bestimmen. Hierbei können auch die Abstände multipler Referenzpunkte 43a, 43b und 43c des entarteten Gewebes 41 zu einem oder mehreren Referenzpunkten 42a und 42b des Tubus 32 berücksichtigt werden. Es ist vorstellbar, dass die anhand der Referenzpunkte 42 und 43 bestimmte Position des Applikators 30 noch nicht zufriedenstellend ist. Der Applikator 30 kann in diesem Fall repositioniert werden und das erfindungsgemäße Verfahren wird wiederholt.

Die Reihenfolge der beschriebenen Verfahrensschritte S1 bis S5 ist nicht als fest anzusehen, sondern kann in Abhängigkeit verschiedener Ausführungsformen des Applikators 30 und Verfahrensanforderungen variieren. Beispielsweise kann die Positionierung des Indikatorelements 34 (S1) entfallen, wenn die Kodierung 33 mit dem Tubus 32 statt dem Führungselement 31 verbunden ist. In diesem Fall kann die Bestimmung der Position des Applikators 30 in der Zielregion 36, wie oben beschrieben, anhand der Kodierung 33 erfolgen. Der Schritt S1 kann ebenso nach der Erfassung des ersten Magnetresonanz-Bilddatensatzes (S2) erfolgen, da dieser auf eine hohe Auflösung des Gewebes in der Zielregion des Patienten 5 ausgelegt ist.

In einem weiteren Beispiel kann der Schritt S2 mit dem Schritt S3 vertauscht sein. Die Reihenfolge der ersten Bildgebungssequenz mit einer hohen Auflösung des Gewebes und der zweiten Bildgebungssequenz mit einer hohen Auflösung des Applikators 30 ist unerheblich, solange der Applikator 30 und/oder das Indikatorelement 34 vor der zweiten Bildgebungssequenz in der Zielregion 36 positioniert sind. Unter dieser Randbedingung kann die zweite Bildgebungssequenz, ebenso wie die erste Bildgebungssequenz, auf eine hohe Auflösung des Gewebes in der Zielregion 36 ausgerichtet sein. Die Position des Applikators 30 wird in diesem Fall in Abhängigkeit einer Differenz zwischen dem ersten Magnetresonanz-Bilddatensatz und dem zweiten Magnetresonanz-Bilddatensatz bestimmt. Es ist auch vorstellbar, dass der Schritt S3 ausfällt, wenn die Kodierung 33 ein Material aufweist, welches gut mittels der ersten Bildgebungssequenz aufgelöst wird.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Applikator (30) zur Positionierung einer Strahlenquelle in einem Patienten (5), wobei der Applikator (30) einen Tubus (32) aufweist und ausgebildet ist, die Strahlenquelle entlang des Tubus (32) in eine Zielregion (36) in einem Körper des Patienten (5) zu leiten,
**dadurch gekennzeichnet, dass** der Applikator (30) ein Indikatorelement (34) aufweist, welches mittels eines medizinischen Bildgebungsverfahrens erfassbar ist, wobei das Indikatorelement (34) mit einem Führungselement (31) verbunden ist und mittels des Führungselements (31) entlang einer Längserstreckung des Tubus (32) in einem Inneren des Tubus (32) verschiebbar ist, wobei das Indikatorelement aus Silikon gefertigt ist und wobei das Führungselement (31) eine Kodierung (33) aufweist, welche mittels eines medizinischen Bildgebungsverfahrens erfassbar ist und ausgelegt ist, einen ersten Applikator (30a) von einem zweiten Applikator (30b) zu unterscheiden.

2. Applikator (30) nach Anspruch 1, wobei die Kodierung (33) und/oder das Indikatorelement (34) und/oder das Führungselement (31) mit magnetischen Feldern wechselwirken und mittels einer Magnetresonanzvorrichtung (18) erfassbar sind.

3. Applikator (30) nach einem der vorhergehenden Ansprüche, wobei das Führungselement (31) eine Markierung (37) aufweist, wobei eine Position des Indikatorelements (34) entlang der Längserstreckung des Tubus (32) anhand einer vorbestimmten Relativposition zwischen der Markierung (37) und einem distalen Ende (38) des Tubus (32) bestimmbar ist.

4. Applikator (30) nach einem der vorhergehenden Ansprüche, wobei der Tubus (32) und/oder das Indikatorelement (34) eine Kodierung (33) aus einem vorbestimmten Material aufweisen.

5. Applikator (30) nach einem der vorhergehenden Ansprüche, wobei die Kodierung (33) als ein individuelles Muster ausgebildet ist, welches über ein medizinisches Bildgebungsverfahren erfassbar ist, wobei das Muster einteilig oder aus disjunkten Abschnitten zusammengesetzt ist.

6. System, umfassend einen ersten Applikator (30a) mit einer ersten Kodierung (33a) nach Anspruch 1 und einen zweiten Applikator mit einer zweiten Kodierung (33b) nach Anspruch 1, wobei der erste Applikator (30a) und der zweite Applikator (30b) anhand der ersten Kodierung (33a) und der zweiten Kodierung (33b) mittels eines medizinischen Bildgebungsverfahrens unterscheidbar sind.

7. Verfahren zur Identifikation eines Applikators (30) zur Positionierung einer Strahlenquelle in einer Zielregion (36) in einem Körper eines Patienten (5) unter Verwendung einer Magnetresonanzvorrichtung (18), wobei der Applikator (30) einen Tubus (32) aufweist und ausgebildet ist, eine Strahlenquelle entlang des Tubus (32) in die Zielregion (36) zu leiten, mit den folgenden Schritten:
• Erfassen eines ersten Magnetresonanz-Bilddatensatzes mittels einer ersten Bildgebungssequenz, wobei erste Bildgebungsparameter der ersten Bildgebungssequenz auf eine hohe Auflösung eines Gewebes in der Zielregion (36) des Patienten (5) gerichtet sind, wobei sich der Applikator (30) in der Zielregion (36) befindet,
• Positionieren eines Indikatorelements (34) mittels eines Führungselements (31) innerhalb des Tubus (32) des Applikators (30), wobei das Indikatorelement (34) aus Silikon gefertigt ist,
• Erfassen eines zweiten Magnetresonanz-Bilddatensatzes, wobei zweite Bildgebungsparameter einer zweiten Bildgebungssequenz auf eine hohe Auflösung des Gewebes in der Zielregion (36) des Patienten gerichtet sind,
• Bestimmen einer Position und einer Kennung des Applikators (30) in der Zielregion (36) anhand des ersten Magnetresonanz-Bilddatensatzes und des zweiten Magnetresonanz-Bilddatensatzes,
• Ausgabe der Position und einer Kennung des Applikators (30).

8. Verfahren nach Anspruch 7, wobei der Applikator (30) eine Kodierung (33) aufweist, welche mittels der Magnetresonanzvorrichtung (18) erfasst wird und zu der Bestimmung der Kennung und/oder der Position des Applikators (30) in der Zielregion (36) des Patienten (5) herangezogen wird.

9. Verfahren nach einem der Ansprüche 7 oder 8, wobei eine Differenz zwischen dem ersten Magnetresonanz-Bilddatensatz und dem zweiten Magnetresonanz-Bilddatensatz zur Bestimmung einer Position des Indikatorelements (34) herangezogen wird.

10. Verfahren nach Anspruch 7, wobei die Bestimmung der Kennung des Applikators (30) anhand einer Fortbewegungsgeschwindigkeit des Indikatorelements (34) erfolgt und die zweite Bildgebungssequenz auf eine hohe Auflösung der Fortbewegungsgeschwindigkeit des Indikatorelements (34) gerichtet ist.

11. Verfahren nach einem der Ansprüche 7 bis 10, wobei in einem Schritt zumindest anhand der Position eines Indikatorelements (34) und/oder der Kodierung (33) des Applikators (30) eine relative Position des Applikators (30) zu einem Gewebe in der Zielregion (36) bestimmt wird.

12. Verfahren nach Anspruch 11, wobei in einem Schritt in Abhängigkeit der Kodierung (33) ein Modell des Applikators (30) in dem ersten Magnetresonanz-Bilddatensatz ausgegeben wird.

## Claims

1. Applicator (30) for positioning a radiation source in a patient (5), wherein the applicator (30) has a tube (32) and is embodied to direct the radiation source along the tube (32) into a target region (36) in a body of the patient (5),
**characterised in that** the applicator (30) has an indicator element (34), which can be detected by means of a medical imaging method, wherein the indicator element (34) is connected to a guide element (31) and can be displaced by means of the guide element (31) along a longitudinal extension of the tube (32) in an interior of the tube (32), wherein the indicator element is manufactured from silicone and wherein the guide element (31) has an encoding (33), which can be detected by means of a medical imaging method and is designed to differentiate a first applicator (30a) from a second applicator (30b).

2. Applicator (30) according to claim 1, wherein the encoding (33) and/or the indicator element (34) and/or the guide element (31) interact with magnetic fields and can be detected by means of a magnetic resonance apparatus (18).

3. Applicator (30) according to one of the preceding claims, wherein the guide element (31) has a marking (37), wherein a position of the indicator element (34) along the longitudinal extension of the tube (32) can be determined on the basis of a predetermined relative position between the marking (37) and a distal end (38) of the tube (32).

4. Applicator (30) according to one of the preceding claims, wherein the tube (32) and/or the indicator element (34) have an encoding (33) made from a predetermined material.

5. Applicator (30) according to one of the preceding claims, wherein the encoding (33) is embodied as an individual pattern, which can be detected via a medical imaging method, wherein the pattern consists of one piece or disjoint sections.

6. System, comprising a first applicator (30a) with a first encoding (33a) according to claim 1 and a second applicator with a second encoding (33b) according to claim 1, wherein the first applicator (30a) and the second applicator (30b) can be differentiated on the basis of the first encoding (33a) and the second encoding (33b) by means of a medical imaging method.

7. Method for identifying an applicator (30) for positioning a radiation source in a target region (36) in a body of a patient (5) with the use of a magnetic resonance apparatus (18), wherein the applicator (30) has a tube (32) and is embodied to direct a radiation source along the tube (32) into the target region (36), with the following steps:
• detecting a first magnetic resonance image data set by means of a first imaging sequence, wherein first imaging parameters of the first imaging sequence are directed towards a high resolution of a tissue in the target region (36) of the patient (5), wherein the applicator (30) is located in the target region (36),
• positioning an indicator element (34) by means of a guide element (31) within the tube (32) of the applicator (30), wherein the indicator element (34) is manufactured from silicone,
• detecting a second magnetic resonance image data set, wherein second imaging parameters of a second imaging sequence are directed towards a high resolution of the tissue in the target region (36) of the patient,
• determining a position and an identifier of the applicator (30) in the target region (36) on the basis of the first magnetic resonance image data set and the second magnetic resonance image data set,
• outputting the position and an identifier of the applicator (30).

8. Method according to claim 7, wherein the applicator (30) has an encoding (33), which is detected by means of the magnetic resonance apparatus (18) and is used for the determining of the identifier and/or the position of the applicator (30) in the target region (36) of the patient (5).

9. Method according to one of claims 7 or 8, wherein a difference between the first magnetic resonance image data set and the second magnetic resonance image data set is used for the determining of a position of the indicator element (34).

10. Method according to claim 7, wherein the determining of the identifier of the applicator (30) takes place on the basis of a forward movement speed of the indicator element (34) and the second imaging sequence is directed towards a high resolution of the forward movement speed of the indicator element (34).

11. Method according to one of claims 7 to 10, wherein in one step a relative position of the applicator (30) in relation to a tissue in the target region (36) is determined at least on the basis of the position of an indicator element (34) and/or the encoding (33) of the applicator (30).

12. Method according to claim 11, wherein in a step a model of the applicator (30) in the first magnetic resonance image data set is output as a function of the encoding (33).

## Revendications

1. Applicateur (30) de mise en position d'une source de rayonnement dans un patient (5), dans lequel l'applicateur (30) a un tube (32) et est constitué pour guider la source de rayonnement le long du tube (32) dans une région (36) cible du corps du patient (5),
**caractérisé en ce que** l'applicateur (30) a un élément (34) indicateur, qui peut être détecté au moyen d'un procédé d'imagerie médicale, dans lequel l'élément (34) indicateur est relié à un élément (31) de guidage et peut se déplacer au moyen de l'élément (31) de guidage le long d'une étendue en longueur du tube (32) à l'intérieur du tube (32), dans lequel l'élément indicateur est en silicone, et dans lequel l'élément (31) de guidage a un codage (33), qui peut être détecté au moyen d'un procédé d'imagerie médicale et qui est conçu pour distinguer un premier applicateur (30a) d'un deuxième applicateur (30b).

2. Applicateur (30) suivant la revendication 1, dans lequel le codage (33) et/ou l'élément (34) indicateur et/ou l'élément (31) de guidage interagissent avec des champs magnétiques et peuvent être détectés au moyen d'un dispositif (18) à résonnance magnétique.

3. Applicateur (30) suivant l'une des revendications précédentes, dans lequel l'élément (31) de guidage a un repère (37), dans lequel une position de l'élément (34) indicateur le long de l'étendue en longueur du tube (32) peut être déterminée à l'aide d'une position relative, déterminée à l'avance, entre le repère (37) et une extrémité (38) distale du tube (32).

4. Applicateur (30) suivant l'une des revendications précédentes, dans lequel le tube (32) et/ou l'élément (34) indicateur ont un codage (33) en un matériau déterminé à l'avance.

5. Applicateur (30) suivant l'une des revendications précédentes, dans lequel le codage (33) est constitué sous la forme d'un motif individuel, qui peut être détecté par un procédé d'imagerie médicale, dans lequel le motif est composé d'une seule pièce ou de parties disjointes.

6. Système comprenant un premier applicateur (30a) ayant un premier codage (33a) suivant la revendication 1 et un deuxième applicateur ayant un deuxième codage (33b) suivant la revendication 1, dans lequel le premier applicateur (30a) et le deuxième applicateur (30b) peuvent être distingués à l'aide du premier codage (33a) et du deuxième codage (33b) au moyen d'un procédé d'imagerie médicale.

7. Procédé d'identification d'un applicateur (30) pour la mise en position d'une source de rayonnement dans une région (36) cible du corps d'un patient (5) en utilisant un dispositif (18) à résonnance magnétique, dans lequel l'applicateur (30) a un tube (32) et est constitué pour guider une source de rayonnement le long du tube (32) dans la région (36) cible, comprenant les stades suivants :
• saisie d'un premier ensemble de données d'image de résonnance magnétique au moyen d'une première séquence d'imagerie, dans lequel les premiers paramètres d'imagerie de la première séquence d'imagerie visent une grande résolution d'un tissu dans la région (36) cible du patient (5), l'applicateur (30) se trouvant dans la région (36) cible,
• mise en position d'un élément (34) d'indicateur au moyen d'un élément (31) de guidage dans le tube (32) de l'applicateur (30), dans lequel l'élément (34) indicateur est en silicone,
• saisie d'un deuxième ensemble de données d'image de résonnance magnétique, dans lequel des deuxièmes paramètres d'imagerie d'une deuxième séquence d'imagerie visent une grande résolution du tissu dans la région (36) cible du patient,
• détermination d'une position et d'une identification de l'applicateur (30) dans la région (36) cible à l'aide du premier ensemble de données d'image de résonnance magnétique et du deuxième ensemble de données d'image de résonnance magnétique,
• émission de la position et d'une identification de l'applicateur (30).

8. Procédé suivant la revendication 7, dans lequel l'applicateur (30) a un codage (33), que l'on détecte au moyen du dispositif (18) de résonnance magnétique et dont on tire parti pour la détermination de l'identification et/ou de la position de l'applicateur (30) dans la région (36) cible du patient (5).

9. Procédé suivant l'une des revendications 7 ou 8, dans lequel on tire parti d'une différence entre le premier ensemble de données d'image de résonnance magnétique et le deuxième ensemble de données d'image de résonnance magnétique pour la détermination d'une position de l'élément (34) indicateur.

10. Procédé suivant la revendication 7, dans lequel la détermination de l'identification de l'applicateur (30) s'effectue à l'aide d'une vitesse de déplacement de l'élément (34) indicateur et la deuxième séquence d'imagerie vise une grande résolution de la vitesse de déplacement de l'élément (34) indicateur.

11. Procédé suivant l'une des revendications 7 à 10, dans lequel dans un stade, on détermine, au moins à l'aide de la position d'un élément (34) indicateur et/ou du codage (33) de l'applicateur (30), une position relative de l'applicateur (30) par rapport à un tissu de la région (36) cible.

12. Procédé suivant la revendication 11, dans lequel dans un stade, on émet en fonction du codage (33) un modèle de l'applicateur (30) dans le premier ensemble de données d'image de résonnance magnétique.
